# EUROPÄISCHE PATENTSCHRIFT

(11) **EP 2 024 310 B1**
(45) Veröffentlichungstag und Bekanntmachung des Hinweises auf die Patenterteilung: **14.03.2012**
(21) Anmeldenummer: 07724812.8
(22) Anmeldetag: 03.05.2007
(51) Int. Cl.: C07C 13/24, C07C 13/26, C07C 13/72, C07C 25/22, C07C 25/24, C07C 33/26, C07C 211/54, C07C 211/58, C07D 307/42, C07D 307/77, C07D 307/93, C07D 209/86, C07D 209/94, C07D 333/78, C07F 5/02, C09B 57/00, C09B 69/10, C07C 13/62

(54) **MATERIALIEN FÜR ORGANISCHE ELEKTROLUMINESZENZVORRICHTUNGEN**
MATERIALS FOR ORGANIC ELECTROLUMINESCENT DEVICES
MATERIAUX POUR DISPOSITIFS ELECTROLUMINESCENTS ORGANIQUES

(30) Priorität: 02.06.2006 DE 102006025846
(43) Veröffentlichungstag der Anmeldung: 18.02.2009
(73) Patentinhaber: Merck Patent GmbH, 64293 Darmstadt (DE)
(72) Erfinder: BUESING, Arne, 65959 Frankfurt am Main (DE); STOESSEL, Philipp, 60487 Frankfurt am Main (DE); HEIL, Holger, 64295 Darmstadt (DE)
(86) Internationale Anmeldenummer: PCT/EP2007/003886
(87) Internationale Veröffentlichungsnummer: WO 2007/140847

(56) Entgegenhaltungen:
- EP-A- 1 217 668
- EP-A1- 1 533 290
- WO-A-03/095445
- US-A1- 2004 147 742
- US-A1- 2005 112 404
- HU N-X ET AL: "5,11-Dihydro-5,11-di-1-naphthylindolo[3,2 -b]carbazole: Atropisomerism in a Novel Hole-Transport Molecule for Organic Light-Emitting Diodes" JOURNAL OF THE AMERICAN CHEMICAL SOCIETY, AMERICAN CHEMICAL SOCIETY, WASHINGTON, DC, US, Bd. 121, 1999, Seiten 5097-5098, XP002331514 ISSN: 0002-7863
- M.T. LEE ET AL: "Stable styrylamine-doped blue rganic electroluminscent device" APPLIED PHYSICS LETTERS, Bd. 85, Nr. 15, 2004, Seiten 3301-3303, XP012062940
- RUIZ M ET AL: "Overcrowded 5,10,15-trisubstituted derivatives: synthesis of 5,10,15-tri(fluorenylidene) truxene" Februar 2004 (2004-02), EUROPEAN JOURNAL OF ORGANIC CHEMISTRY, WILEY-VCH VERLAG, WEINHEIM, DE, PAGE(S) 858-866 , XP002350737 ISSN: 1434-193X das ganze Dokument

## Beschreibung

Die vorliegende Erfindung betrifft organische Halbleiter und deren Verwendung in organischen elektronischen Vorrichtungen.

Organische Halbleiter werden für eine Reihe verschiedenartiger Anwendungen, die im weitesten Sinne der Elektronikindustrie zugerechnet werden können, entwickelt. Der Aufbau organischer Elektrolumineszenzvorrichtungen (OLEDs), in denen diese organischen Halbleiter als funktionelle Materialien eingesetzt werden, ist beispielsweise in US 4539507, US 5151629, EP 0676461 und WO 98/27136 beschrieben. Allerdings zeigen diese Vorrichtungen noch erhebliche Probleme, die einer dringenden Verbesserung bedürfen:
1. Die üblicherweise verwendeten Verbindungen weisen kein ausreichend tiefes LUMO (niedrigstes unbesetztes Molekülorbital) auf. Verbindungen mit niedrigerem LUMO werden für leichtere Elektroneninjektion und damit für eine Verringerung der Betriebsspannung benötigt.
2. In Systemen gemäß dem Stand der Technik werden in der emittierenden Schicht überlicherweise ein oder mehrere Dotanden in einem Hostmaterial verwendet. Es wäre sinnvoll, Verbindungen zu haben, die als Reinsubstanz in der emittierenden Schicht zu verwenden sind, da dies eine technische Vereinfachung bei der Device-Herstellung darstellt.
3. Die Lebensdauer der organischen Elektrolumineszenzvorrichtung ist noch nicht ausreichend für langlebige hochwertige Anwendungen.
4. Die thermische Stabilität vieler organischer Verbindungen, die derzeit in organischen Elektrolumineszenzvorrichtungen verwendet werden, ist ungenügend, so dass sich erhebliche Probleme sowohl bei der Reinigung des Materials durch Massensublimation wie auch beim Aufbringen des Materials durch thermische Verdampfung ergeben. Dies gilt insbesondere für Verbindungen, welche Styrylaminogruppen enthalten, wie sie häufig als blau emittierende Verbindungen verwendet werden.

Für fluoreszierende OLEDs werden gemäß dem Stand der Technik vor allem kondensierte Aromaten, insbesondere Anthracen- oder Pyrenderivate, als Host-Materialien vor allem für blau emittierende Elektrolumineszenzvorrichtungen verwendet, z. B. 9,10-Bis(2-naphthyl)anthracen (US 5935721). In WO 03/095445 und in CN 1362464 werden 9,10-Bis(1-naphthyl)anthracen-Derivate für die Verwendung in OLEDs offenbart. Weitere Anthracenderivate sind in WO 01/076323, in WO 01/021729, in WO 04/013073, in WO 04/018588, in WO 03/087023 oder in WO 04/018587 offenbart. Host-Materialien, basierend auf Arylsubstituierten Pyrenen und Chrysenen, werden in WO 04/016575 offenbart. Es ist für hochwertige Anwendungen notwendig, verbesserte Host-Materialien zur Verfügung zu haben.

Als Stand der Technik bei blau emittierenden Verbindungen kann die Verwendung von Arylvinylaminen genannt werden (z. B. WO 04/013073, WO 04/016575, WO 04/018587). Diese Verbindungen sind allerdings thermisch instabil und lassen sich nicht unzersetzt verdampfen, was einen hohen technischen Aufwand für die Synthese und die OLED-Herstellung erfordert und somit einen technischen Nachteil darstellt. Einen weiteren Nachteil stellt die Emissionsfarbe dieser Verbindungen dar: Während im Stand der Technik mit diesen Verbindungen tiefblaue Emission (CIE-y-Koordinaten im Bereich von 0.15-0.18) beschrieben wird, konnten diese Farbkoordinaten nicht in einfachen Vorrichtungen gemäß dem Stand der Technik reproduziert werden. Im Gegenteil erhält man hier grünblaue Emission. Es ist nicht offensichtlich, wie mit diesen Verbindungen tatsächlich blaue Emission erzeugt werden kann. Es ist daher für hochwertige Anwendungen notwendig, verbesserte Emitter besonders im Bezug auf Device- und Sublimationsstabilität sowie Emissionsfarbe zur Verfügung zu haben.

In phosphoreszierenden OLEDs wird als Matrixmaterial häufig 4,4'-Bis-(N-carbazolyl)biphenyl (CBP) verwendet. Die Nachteile sind kurze Lebensdauern der mit ihnen hergestellten Devices und häufig hohe Betriebsspannungen, die zu geringen Leistungseffizienzen führen. Außerdem weist CBP eine nicht ausreichend hohe Glasübergangstemperatur auf. Des Weiteren hat sich gezeigt, dass CBP für blau emittierende Elektrolumineszenzvorrichtungen ungeeignet ist, was in einer schlechten Effizienz resultiert. Außerdem ist der Aufbau der Devices mit CBP komplex, da zusätzlich eine Lochblockierschicht und eine Elektronentransportschicht verwendet werden müssen. Verbesserte Triplett-Matrixmaterialien, basierend auf Ketoverbindungen, sind in WO 04/093207 beschrieben. Für die besten der dort beschriebenen Matrixmaterialien werden jedoch für die Synthese giftige anorganische Cyanide offenbart, so dass die Herstellung dieser Materialien ökologisch bedenklich ist. Von anderen der dort beschriebenen Matrixmaterialien ist die Glasübergangstemperatur noch nicht zufrieden stellend.

Als Elektronentransportverbindung in organischen Elektrolumineszenzvorrichtungen wird meist AlQ₃ (Aluminium-tris-hydroxychinolinat) verwendet (US 4539507). Dieses hat mehrere Nachteile: Es lässt sich nicht rückstandsfrei aufdampfen, da es sich bei der Sublimationstemperatur teilweise zersetzt, was insbesondere für Produktionsanlagen ein großes Problem darstellt. Ein weiterer Nachteil ist die starke Hygroskopie von AlQ₃, ebenso wie die niedrige Elektronenbeweglichkeit, was zu höheren Spannungen und damit zu einer niedrigeren Leistungseffizienz führt. Um Kurzschlüsse im Display zu vermeiden, würde man gern die Schichtdicke erhöhen; dies ist mit AlQ₃ wegen der geringen Ladungsträgerbeweglichkeit und der daraus resultierenden Spannungserhöhung nicht möglich. Als sehr ungünstig erweist sich weiterhin die Eigenfarbe von AlQ₃ (im Feststoff gelb), die gerade bei blauen OLEDs durch Reabsorption und schwache Reemission zu Farbverschiebungen führen kann. Hier sind blaue OLEDs nur mit starken Effizienz- bzw. Farborteinbußen darstellbar. Trotz der genannten Nachteile stellt AlQ₃ in OLEDs bislang immer noch den besten Kompromiss für die verschiedenartigen Anforderungen an ein Elektronentransportmaterial in OLEDs dar.

Es besteht also weiterhin Bedarf an verbesserten Materialien, insbesondere Host-Materialien für blau fluoreszierende Emitter und Triplett-Emitter, aber auch emittierende Verbindungen, insbesondere blau emittierende Verbindungen, Lochtransportmaterialien und Elektronentransportmaterialien, die thermisch stabil sind, die in organischen elektronischen Vorrichtungen zu guten Effizienzen und gleichzeitig zu hohen Lebensdauern führen, die bei der Herstellung und beim Betrieb der Vorrichtung zu reproduzierbaren Ergebnissen führen, die synthetisch einfach und in hohen Ausbeuten zugänglich sind und die hohe thermische Stabilität aufweisen.

Überraschend wurde gefunden, dass Verbindungen, in denen eine Phenylengruppe in para-Position mit zwei Naphthylgruppen verknüpft ist, wobei die Naphthylgruppen über die 1- oder die 2-Position mit der Phenylengruppe verknüpft sein können, und in denen noch zusätzlich jeweils mindestens eine Brücke zwischen der Phenylengruppe und den beiden Naphthylgruppen existiert, und heterocyclische Derivate dieser Verbindungen sich sehr gut für die Verwendung in organischen Elektrolumineszenzvorrichtungen eignen. Diese Verbindungen weisen eine hohe thermische Stabilität auf. Mit diesen Materialien ist weiterhin eine Steigerung der Effizienz und der Lebensdauer der organischen elektronischen Vorrichtung im Vergleich zu Materialien gemäß dem Stand der Technik möglich. Weiterhin sind diese Materialien sehr gut für die Verwendung in organischen elektronischen Vorrichtungen geeignet, da sie eine sehr hohe Glasübergangstemperatur aufweisen. Diese Materialien und deren Verwendung in organischen elektronischen Vorrichtungen, insbesondere in organischen Elektrolumineszenzvorrichtungen sind daher der Gegenstand der vorliegenden Erfindung.

Gegenstand der Erfindung sind Verbindungen gemäß Formel (1) bis Formel (4), 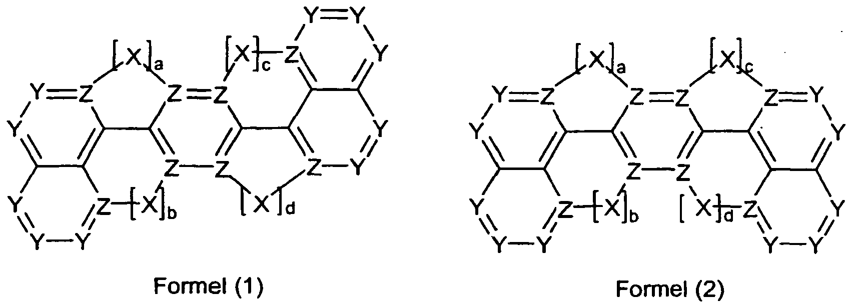 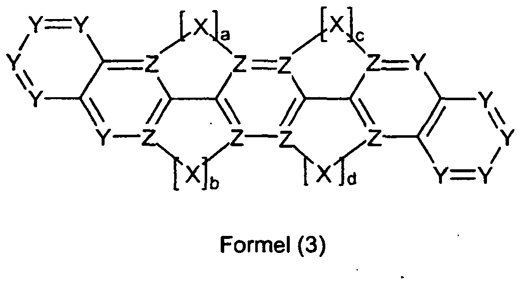 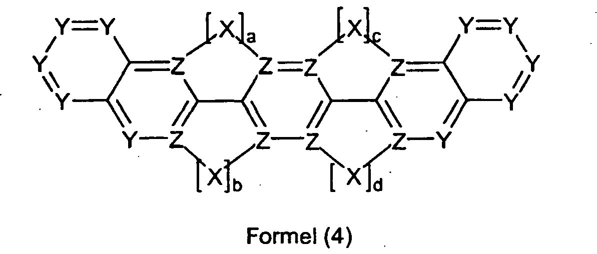 wobei für die Symbole und Indizes gilt:
- Y: ist bei jedem Auftreten gleich oder verschieden CR¹ oder N;
- Z: ist gleich C, falls an die Gruppe Z eine Brücke X gebunden ist, und ist gleich Y, falls an die Gruppe Z keine Brücke X gebunden ist;
- X: ist bei jedem Auftreten gleich oder verschieden eine bivalente Brücke, ausgewählt aus B(R¹), C(R¹)₂, Si(R¹)₂, C=O, C=NR¹, C=C(R¹)₂, O, S, S=O, SO₂, N(R¹), P(R¹) und P(=O)R¹;
- R¹: ist bei jedem Auftreten gleich oder verschieden H, F, Cl, Br, I, N(Ar)₂, C(=O)Ar, P(=O)Ar₂, S(=O)Ar, S(=O)₂Ar, CR²=CR²Ar, CN, NO₂, Si(R²)₃, B(OR²)₂, OSO₂R², eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R² substituiert sein kann und wobei eine oder mehrere nicht benachbarte CH₂-Gruppen durch R²C=CR², C≡C, Si(R²)₂, Ge(R²)₂, Sn(R²)₂, C=O, C=S, C=Se, C=NR², P(=O)(R²), SO, SO₂, NR²,O, S oder CONR² ersetzt sein können und wobei ein oder mehrere H-Atome durch F, Cl, Br, I, CN oder NO₂ ersetzt sein können, oder ein aromatisches oder heteroaromatisches Ringsystem mit 5 bis 40 aromatischen Ringatomen, das jeweils durch einen oder mehrere Reste R² substituiert sein kann, oder eine Aryloxy- oder Heteroaryloxygruppe mit 5 bis 40 aromatischen Ringatomen, die durch einen oder mehrere Reste R² substituiert sein kann, oder eine Kombination dieser Systeme; dabei können zwei oder mehrere benachbarte Substituenten R¹ auch miteinander ein mono- oder polycyclisches aliphatisches oder aromatisches Ringsystem bilden;
- Ar: ist bei jedem Auftreten gleich oder verschieden ein aromatisches oder heteroaromatisches Ringsystem mit 5-30 aromatischen Ringatomen, das mit einem oder mehreren nicht-aromatischen Resten R¹ substituiert sein kann; dabei können auch zwei Reste Ar am selben Stickstoff- oder Phosphoratom durch eine Einfachbindung oder eine Brücke X miteinander verknüpft sein;
- R²: ist bei jedem Auftreten gleich oder verschieden H oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten R² auch miteinander ein mono- oder polycyclisches aliphatisches oder aromatisches Ringsystem bilden;
- a, b, c, d: ist bei jedem Auftreten gleich oder verschieden 0 oder 1, mit der Maßgabe, dass a + b = 1 oder 2 und c + d = 1 oder 2 ist, wobei a = 0 bzw. b = 0 bzw. c = 0 bzw. d = 0 jeweils bedeutet, dass die entsprechende Brücke X nicht vorhanden ist;
ausgenommen sind die folgenden Verbindungen:

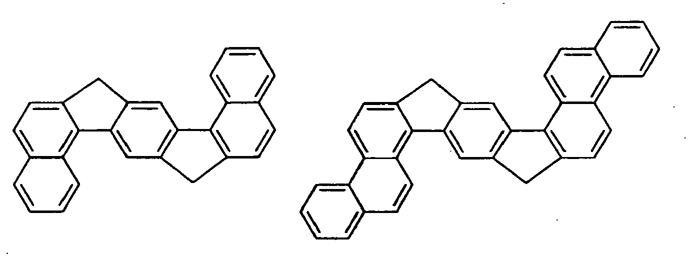

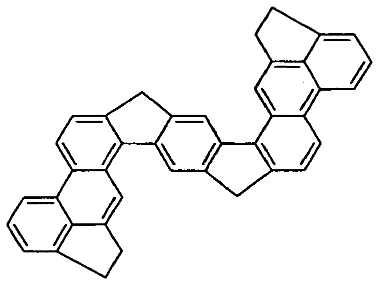

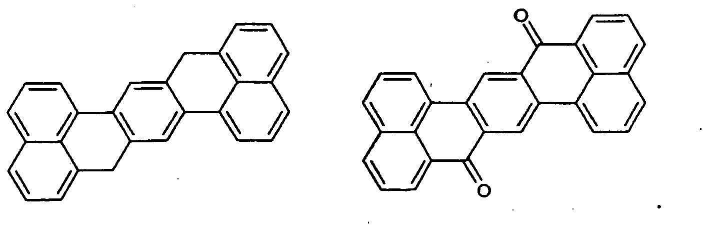

Bevorzugt weisen die Verbindungen gemäß Formel (1) bis Formel (4) eine Glasübergangstemperatur T_{g} von größer als 70 °C auf, besonders bevorzugt größer als 100 °C, ganz besonders bevorzugt größer als 130 °C. Unter benachbarten Resten R¹ bzw. R² im Sinne dieser Erfindung werden Reste verstanden, die entweder am selben Kohlenstoffatom bzw. am selben Heteroatom gebunden sind oder die an benachbarten Kohlenstoffatomen bzw. Heteroatomen gebunden sind.

Eine Arylgruppe im Sinne dieser Erfindung enthält 6 bis 40 C-Atome; eine Heteroarylgruppe im Sinne dieser Erfindung enthält 2 bis 40 C-Atome und mindestens 1 Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Pyren, Chinolin, Isochinolin, etc., verstanden.

Ein aromatisches Ringsystem im Sinne dieser Erfindung enthält 6 bis 40 C-Atome im Ringsystem. Ein heteroaromatisches Ringsystem im Sinne dieser Erfindung enthält 2 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit (bevorzugt weniger als 10 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl,
2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden besonders bevorzugt Methoxy, Trifluormethoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 5 - 40 aromatischen Ringatomen, welches noch jeweils mit den oben genannten Resten R substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothlophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol. Bevorzugt sind Strukturen gemäß Formel (1) bis (4), bei denen a + b = 1 und c + d = 1 ist.

Bevorzugt sind die Verbindungen gemäß den Formeln (1) bis (4) ausgewählt aus den Strukturen gemäß den Formeln (5), (6), (7), (8), (9), (10), (11), (12), (13), (14), (15) oder (16), 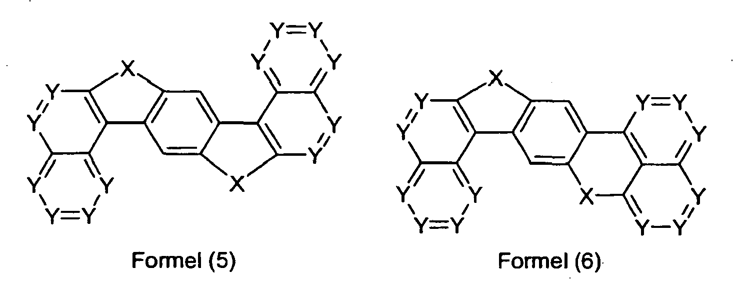 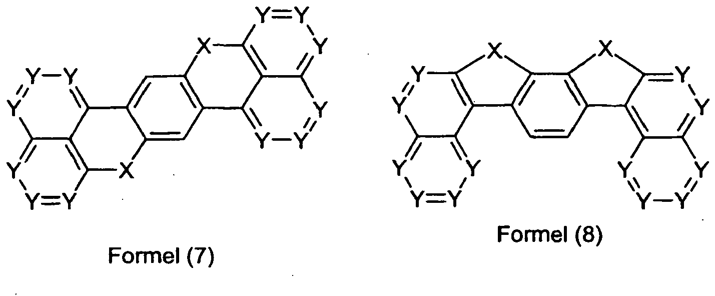 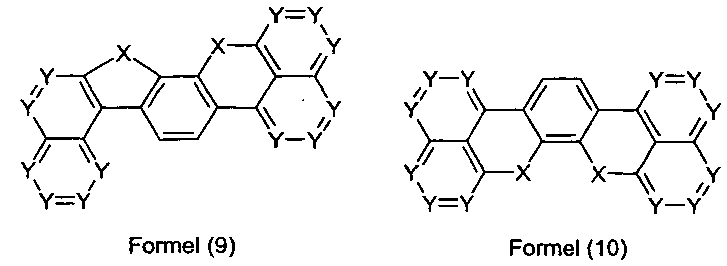 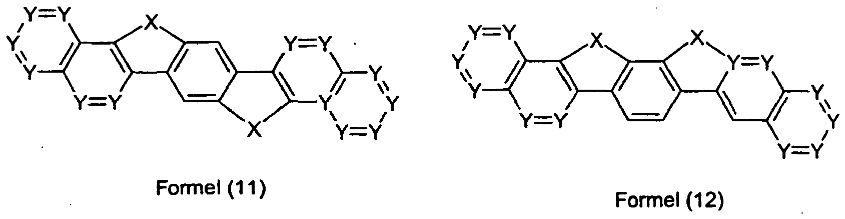 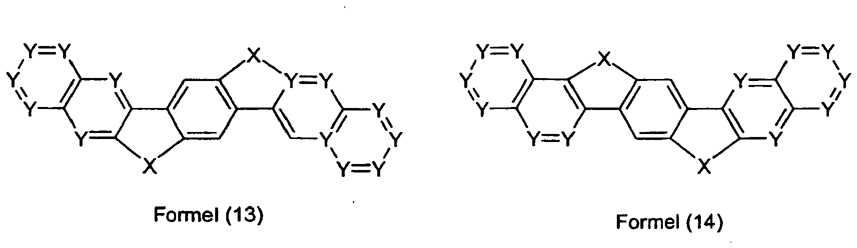 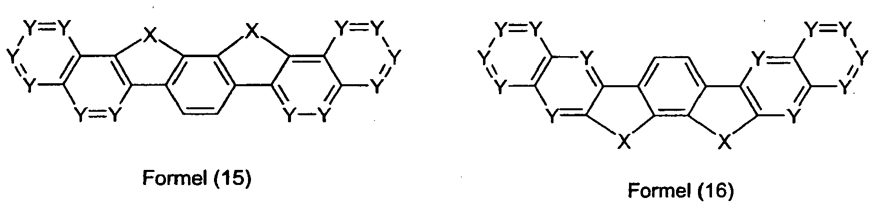 wobei die Symbole X und Y dieselbe Bedeutung haben, wie oben beschrieben.

Bevorzugt sind weiterhin Verbindungen gemäß Formel (1) bis Formel (16), in denen das Symbol Y insgesamt 0, 1, 2, 3 oder 4 Mal für Stickstoff steht, wobei die anderen Symbole Y für CR¹ stehen. Besonders bevorzugt sind Verbindungen gemäß Formel (1) bis Formel (16), in denen das Symbol Y insgesamt 0, 1 oder 2 Mal für Stickstoff steht. In einer besonders bevorzugten Ausführungsform der Erfindung steht das Symbol Y für CR¹.

In einer ganz besonders bevorzugten Ausführungsform sind die Strukturen gemäß Formel (1) bis (4) ausgewählt aus den Formeln (5a), (6a), (7a), (8a), (9a), (10a) und (11a), 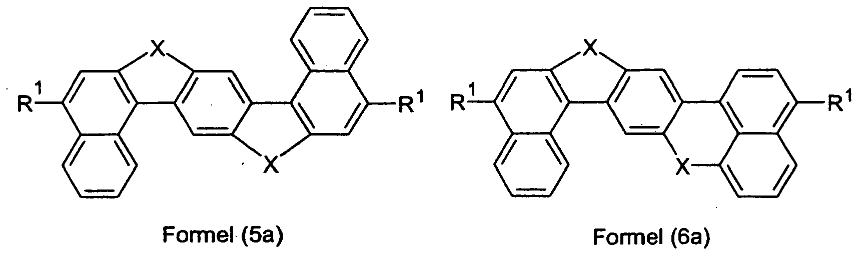 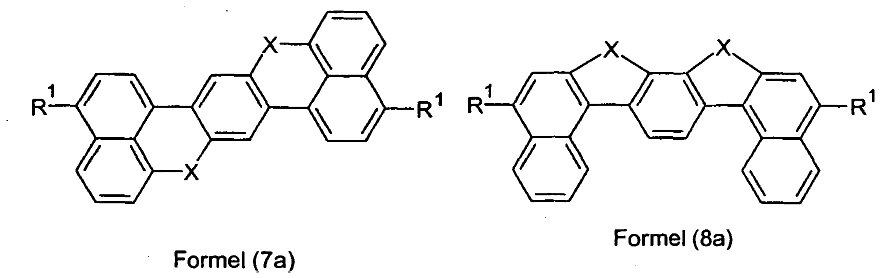 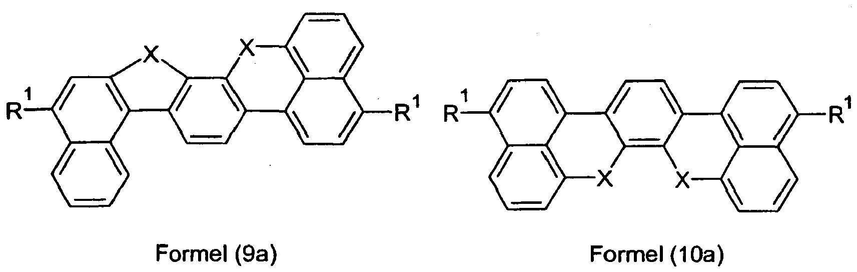 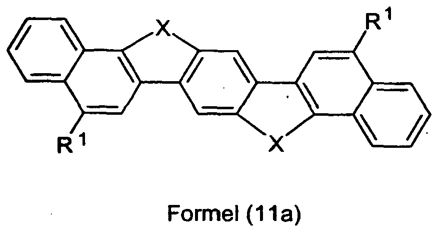 wobei die Symbole X und R¹ dieselbe Bedeutung haben, wie oben beschrieben.

Besonders bevorzugt sind in den Strukturen gemäß den Formeln (5a) bis (11a) jeweils beide Reste R¹ ungleich Wasserstoff.

Bevorzugt sind weiterhin Verbindungen gemäß Formel (1) bis (16) bzw. (5a) bis (11a), in denen das Symbol R¹ gleich oder verschieden bei jedem Auftreten für H, F, Br, C(=O)Ar, P(=O)Ar₂, CR²=CR²Ar, eine geradkettige Alkylgruppe mit 1 bis 5 C-Atomen oder verzweigte Alkylgruppe mit 3 bis 5 C-Atomen, wobei eine oder mehrere nicht benachbarte CH₂-Gruppen durch -R²C=CR²-, -C≡C- oder -O- ersetzt sein können und wobei ein oder mehrere H-Atome durch F ersetzt sein können, oder eine Arylgruppe mit 6 bis 16 C-Atomen oder Heteroarylgruppe mit 2 bis 16 C-Atomen oder eine Spirobifluorengruppe, die jeweils mit einem oder mehreren Resten R² substituiert sein können, oder eine Kombination aus zwei oder drei dieser Systeme steht. Besonders bevorzugte Reste R¹ sind gleich oder verschieden bei jedem Auftreten H, F, Br, C(=O)Ar, P(=O)Ar₂, Methyl, Ethyl, iso-Propyl, tert-Butyl, wobei ein oder mehrere H-Atome durch F ersetzt sein können, oder eine Arylgruppe mit 6 bis 14 C-Atomen oder eine Spirobifluorengruppe, die jeweils mit einem oder mehreren Resten R² substituiert sein können, oder eine Kombination aus zwei dieser Systeme. Bei Einbau in Polymere, Oligomere oder Dendrimere und bei Verbindungen, die aus Lösung verarbeitet werden, sind auch lineare oder verzweigte Alkylketten mit bis zu 10 C-Atomen bevorzugt. Brom als Substituent ist vor allem für die Verwendung dieser Verbindung als Zwischenstufe zur Herstellung anderer erfindungsgemäßer Verbindungen oder zur Verwendung als Monomer zur Herstellung von Polymeren bevorzugt.

Bevorzugt sind weiterhin Verbindungen der Formel (1) bis (16) bzw. (5a) bis (11a), in denen mindestens ein Symbol R¹ für eine Gruppe N(Ar)₂ der Formel (17) oder (18) steht, 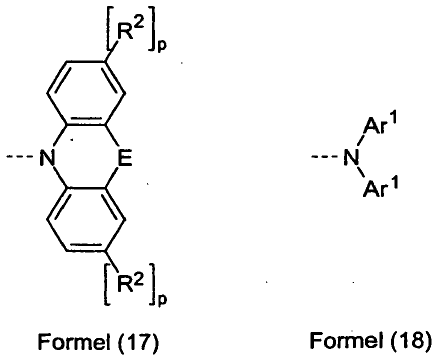 wobei R² die oben aufgeführte Bedeutung hat und weiterhin gilt:
- E: steht für eine Einfachbindung, O, S, N(R²) oder C(R²)₂;
- Ar¹: ist gleich oder verschieden bei jedem Auftreten eine Aryl- oder Heteroarylgruppe mit 5 bis 20 aromatischen Ringatomen oder eine Triarylamingruppe mit 15 bis 30 aromatischen Ringatomen, welche jeweils mit einem oder mehreren Resten R² substituiert sein kann, bevorzugt eine Aryl- oder Heteroarylgruppe mit 6 bis 14 aromatischen Ringatomen oder eine Triarylamingruppe mit 18 bis 22 aromatischen Ringatomen, welche jeweils mit einem oder mehreren Resten R² substituiert sein kann;
- p: ist bei jedem Auftreten gleich oder verschieden 0 oder 1.

Besonders bevorzugt sind Verbindungen der oben aufgeführten Formeln (5a) bis (11a), in denen die Reste R¹ für eine Gruppe der Formel (17) bzw. Formel (18) stehen.

Besonders bevorzugt steht Ar¹ gleich oder verschieden für Phenyl, ortho-, meta- oder para-Tolyl, para-Fluorphenyl, 1-Naphthyl, 2-Naphthyl, Triphenylamin, Naphthyldiphenylamin oder Dinaphthylphenylamin.

Bevorzugt sind weiterhin Verbindungen gemäß Formel (1) bis (16) bzw. (5a) bis (11a), in denen die Symbole X bei jedem Auftreten gleich oder verschieden eine bivalente Brücke sind, ausgewählt aus C(R¹)₂, C=O, C=NR¹, O, S, S=O, SO₂, N(R¹), P(R¹) oder P(=O)R¹. Besonders bevorzugt sind Verbindungen gemäß Formel (1) bis (16) bzw. (5a) bis (11a), in denen die Symbole X bei jedem Auftreten gleich oder verschieden ausgewählt sind aus C(R¹)₂, N(R¹), P(R¹) und P(=O)(R¹), ganz besonders bevorzugt C(R¹)₂ oder N(R¹), insbesondere C(R¹)₂. Dabei sei hier nochmals explizit darauf hingewiesen, dass hier auch mehrere benachbarte Reste R¹ miteinander ein aromatisches oder aliphatisches Ringsystem bilden können. Wenn mehrere Reste R¹ an einer Gruppe C(R¹)₂ miteinander ein Ringsystem bilden, führt dies zu Spirostrukturen. Die Ausbildung derartiger Spirostrukturen durch Bildung von Ringsystemen zwischen zwei Gruppen R¹ an C(R¹)₂ ist eine weitere bevorzugte Ausführungsform der Erfindung. Dies gilt insbesondere, wenn R¹ für eine substituierte oder unsubstituierte Phenylgruppe steht und die beiden Phenylgruppen zusammen mit der Brücke X ein Ringsystem bilden.

Bevorzugte Reste R¹, die an die Brücken X gebunden sind, sind gleich oder verschieden und sind ausgewählt aus H, geradkettigen Alkylgruppen mit 1 bis 5 C-Atomen oder verzweigten Alkylgruppen mit 3 bis 5 C-Atomen, wobei jeweils eine oder mehrere nicht benachbarte CH₂-Gruppen durch -R²C=CR²-, -C≡C- oder -O- ersetzt sein können und wobei ein oder mehrere H-Atome durch F ersetzt sein können, oder Arylgruppen mit 6 bis 16 C-Atomen oder Heteroarylgruppen mit 2 bis 16 C-Atomen, die jeweils mit einem oder mehreren Resten R² substituiert sein können, oder eine Kombination aus zwei oder drei dieser Systeme; dabei können zwei der Reste R¹, die an dasselbe Brückenatom gebunden sind, auch miteinander ein Ringsystem bilden. Besonders bevorzugte Reste R¹, die an die Brücken X gebunden sind, sind gleich oder verschieden und sind ausgewählt aus Methyl, Ethyl, iso-Propyl, tert-Butyl, wobei jeweils ein oder mehrere H-Atome durch F ersetzt sein können, oder Arylgruppen mit 6 bis 14 C-Atomen, die mit einem oder mehreren Resten R² substituiert sein können, oder eine Kombination aus zwei dieser Systeme; dabei können zwei der Reste R¹, die an dasselbe Brückenatom gebunden sind, auch miteinander ein Ringsystem bilden. Bei Einbau in Polymere, Oligomere oder Dendrimere und bei Verbindungen, die aus Lösung verarbeitet werden, sind auch lineare oder verzweigte Alkylketten mit bis zu 10 C-Atomen bevorzugt.

Weiterhin bevorzugt sind symmetrische und symmetrisch substituierte Verbindungen, also Verbindungen, in denen die Symbole X gleich sind. Weiterhin bevorzugt sind in den Strukturen der Formeln (5a) bis (11a) die Substituenten R¹ gleich gewählt.

Beispiele für bevorzugte Verbindungen gemäß Formel (1) bis Formel (16) bzw. (5a) bis (11a) sind die im Folgenden abgebildeten Strukturen (1) bis (132).

| | |
|---|---|
| 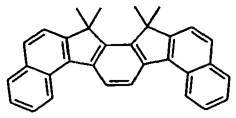 | 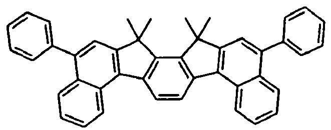 |
| (1) | (2) |
| 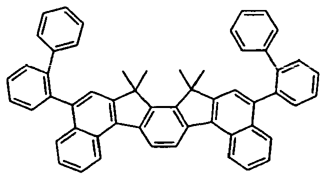 | 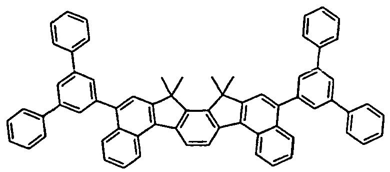 |
| (3) | (4) |
| 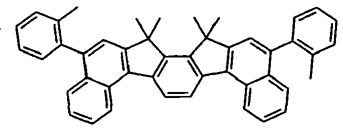 | 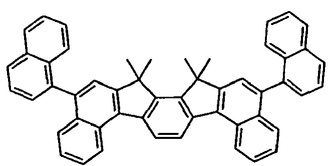 |
| (5) | (6) |
| 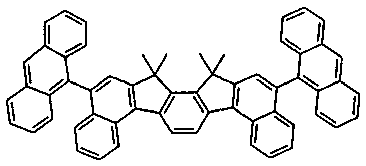 | 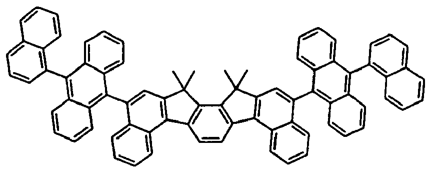 |
| (7) | (8) |
| 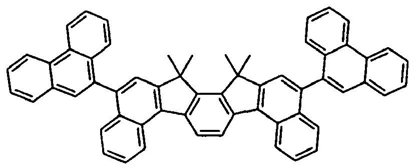 | 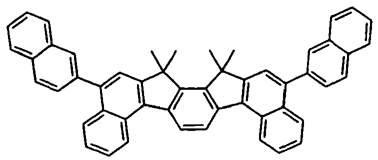 |
| (9) | (10) |
| 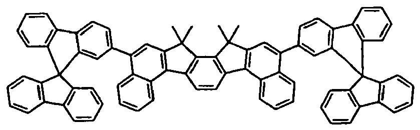 | 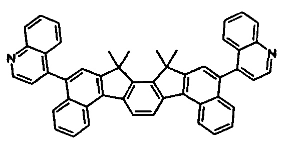 |
| (11) | (12) |
| 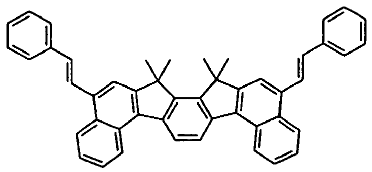 | 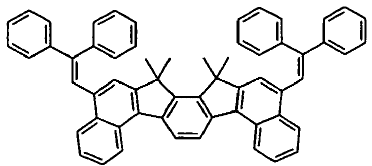 |
| (13) | (14) |
| 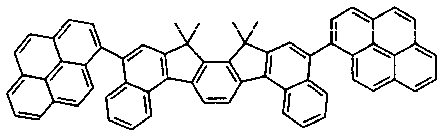 | 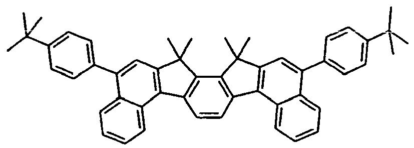 |
| (15) | (16) |
| 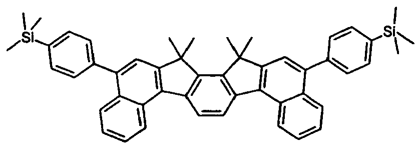 | 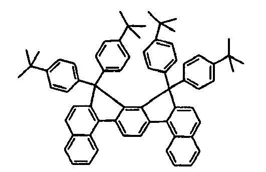 |
| (17) | (18) |
| 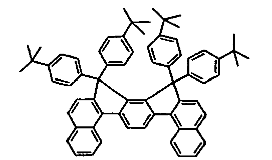 | 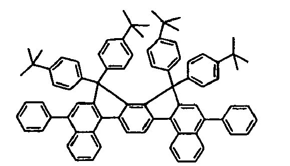 |
| (19) | (20) |
| 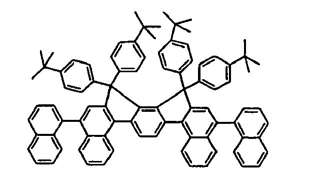 | 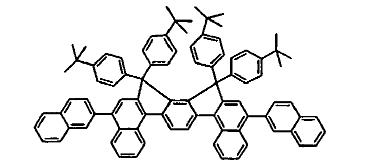 |
| (21) | (22) |
| 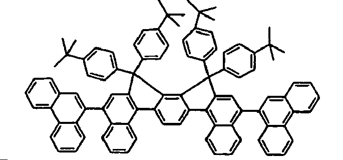 | 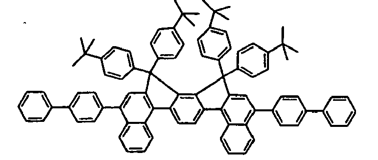 |
| (23) | (24) |
| 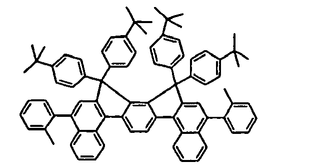 | 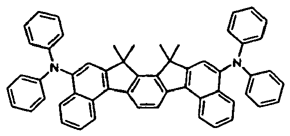 |
| (25) | (26) |
| 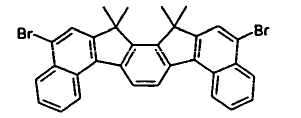 | 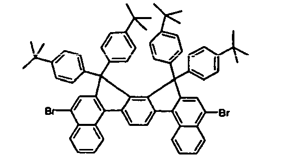 |
| (27) | (28) |
| 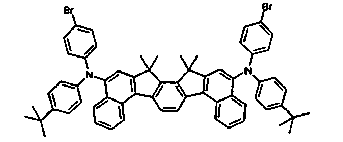 | 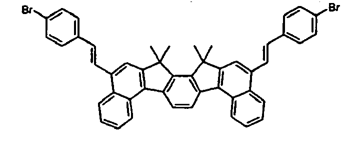 |
| (29) | (30) |
| 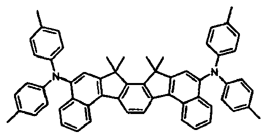 | 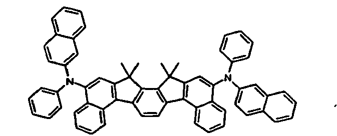 |
| (31) | (32) |
| 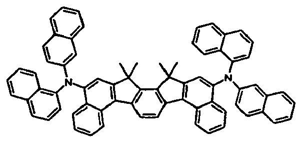 | 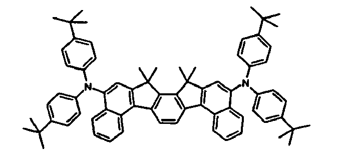 |
| (33) | (34) |
| 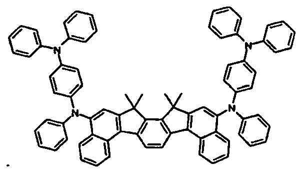 | 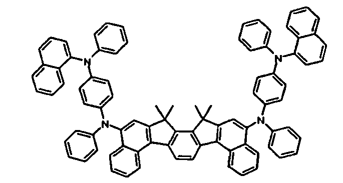 |
| (35) | (36) |
| 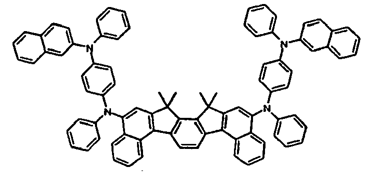 | 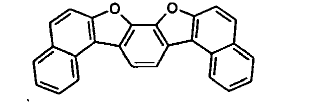 |
| (37) | (38) |
| 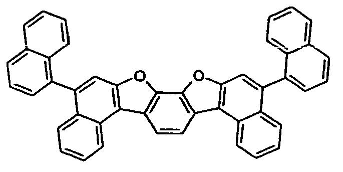 | 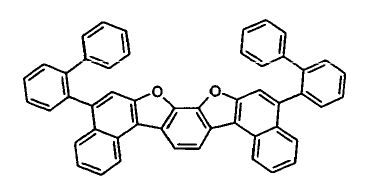 |
| (39) | (40) |
| 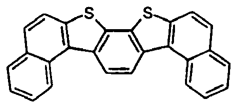 | 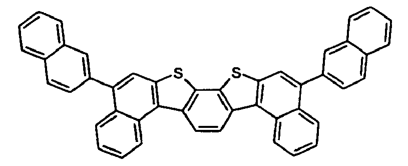 |
| (41) | (42) |
| 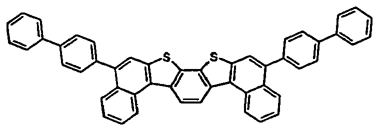 | 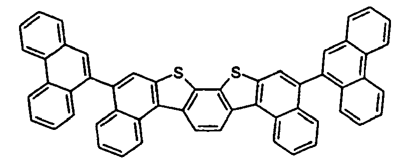 |
| (43) | (44) |
| 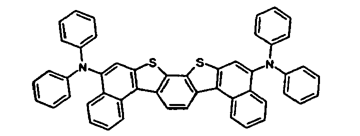 | 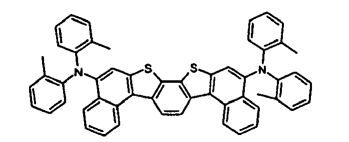 |
| (45) | (46) |
| 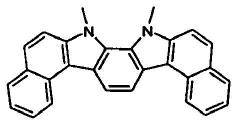 | 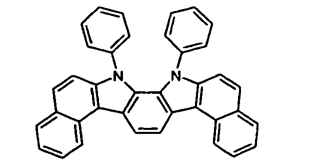 |
| (47) | (48) |
| 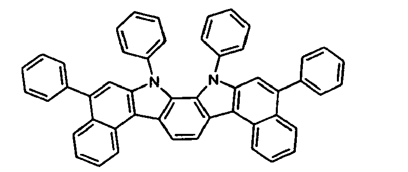 | 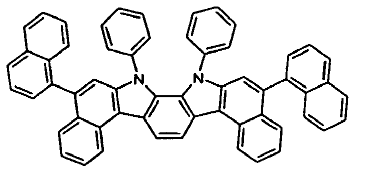 |
| (49) | (50) |
| 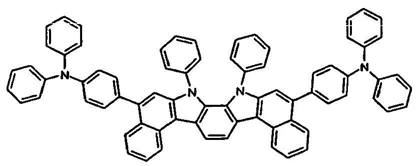 | 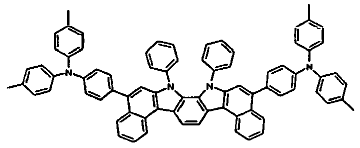 |
| (51) | (52) |
| 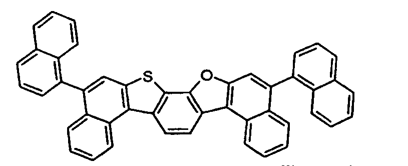 | 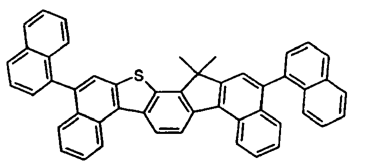 |
| (53) | (54) |
| 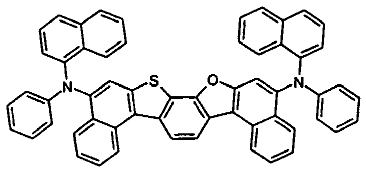 | 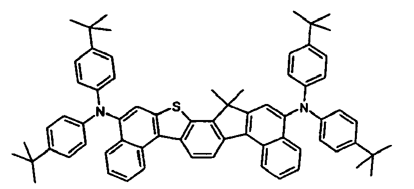 |
| (55) | (56) |
| 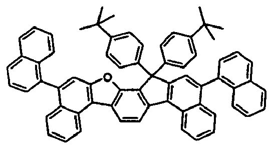 | 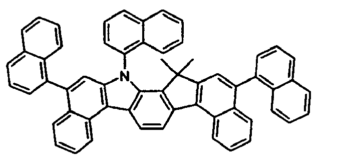 |
| (57) | (58) |
| 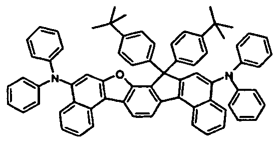 | 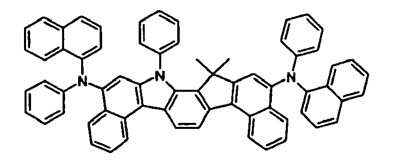 |
| (59) | (60) |
| 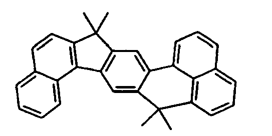 | 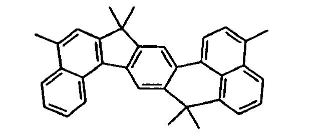 |
| (61) | (62) |
| 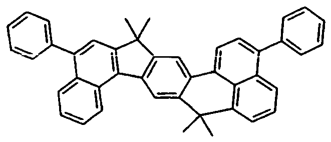 | 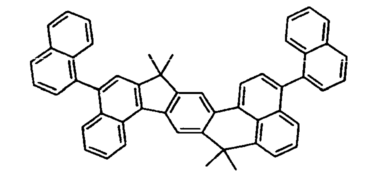 |
| (63) | (64) |
| 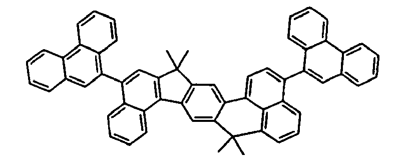 | 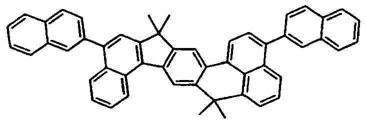 |
| (65) | (66) |
| 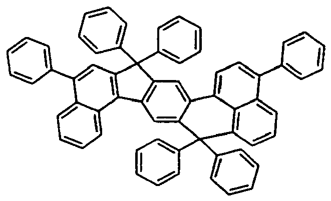 | 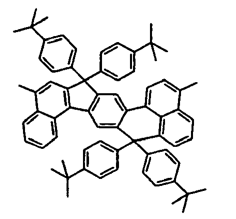 |
| (67) | (68) |
| 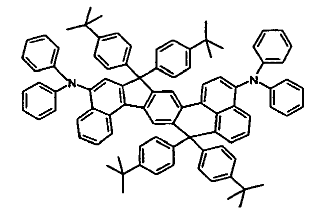 | 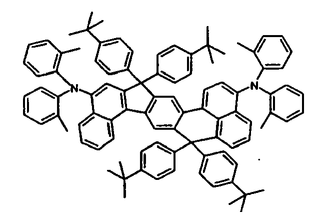 |
| (69) | (70) |
| 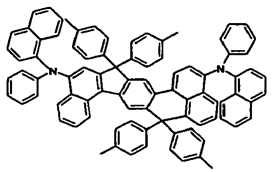 | 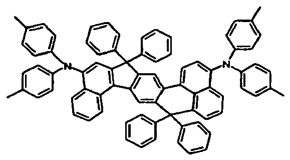 |
| (71) | (72) |
| 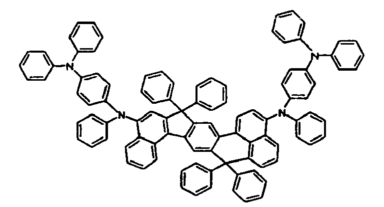 | 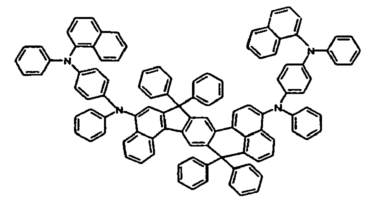 |
| (73) | (74) |
| 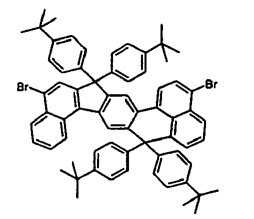 | 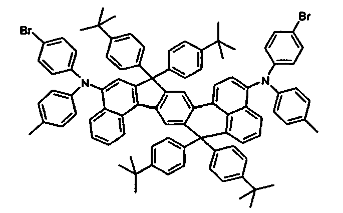 |
| (75) | (76) |
| 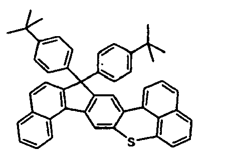 | 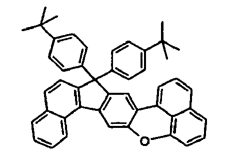 |
| (77) | (78) |
| 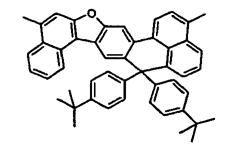 | 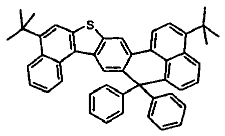 |
| (79) | (80) |
| 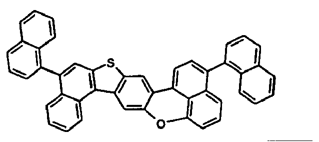 | 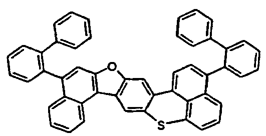 |
| (81) | (82) |
| 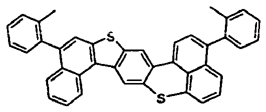 | 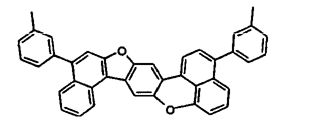 |
| (83) | (84) |
| 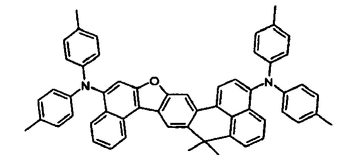 | 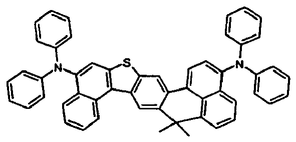 |
| (85) | (86) |
| 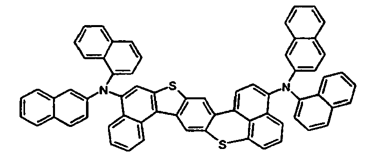 | 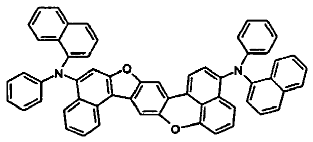 |
| (87) | (88) |
| 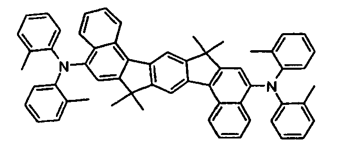 | 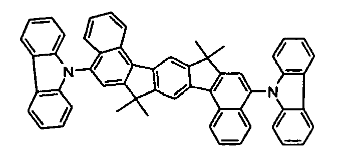 |
| (89) | (90) |
| 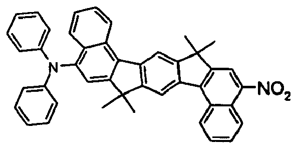 | 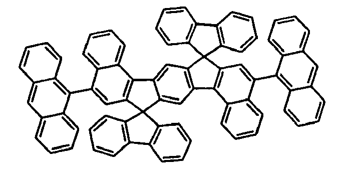 |
| (91) | (92) |
| 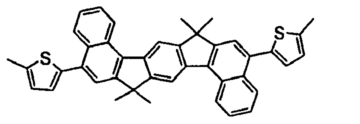 | 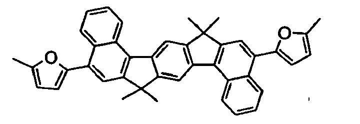 |
| (93) | (94) |
| 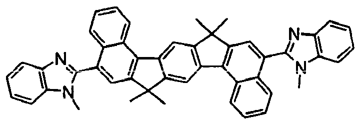 | 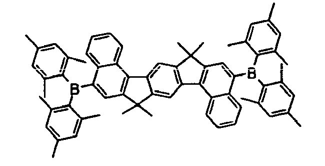 |
| (95) | (96) |
| 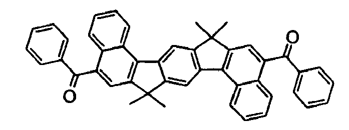 | 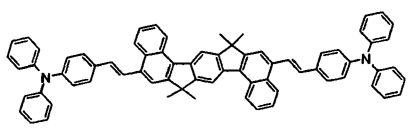 |
| (97) | (98) |
| 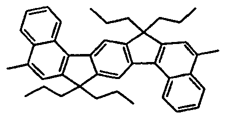 | 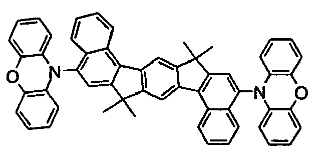 |
| (99) | (100) |
| 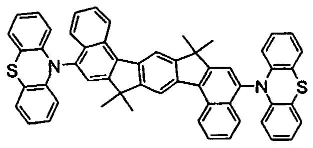 | 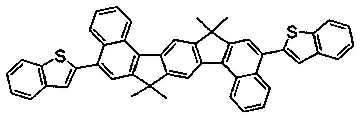 |
| (101) | (102) |
| 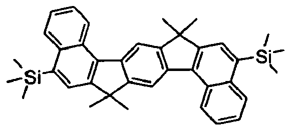 | 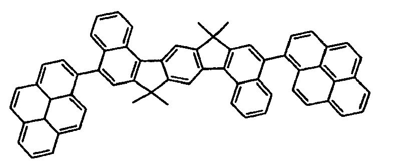 |
| (103) | (104) |
| 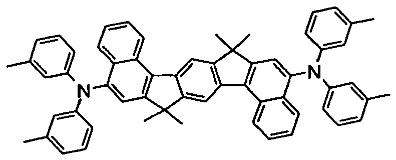 | 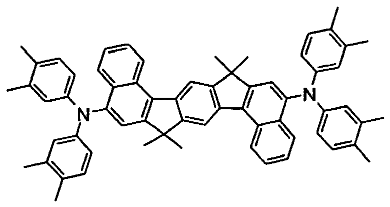 |
| (105) | (106) |
| 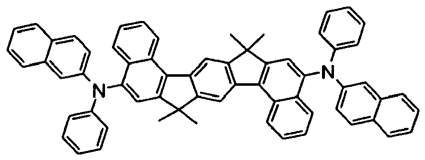 | 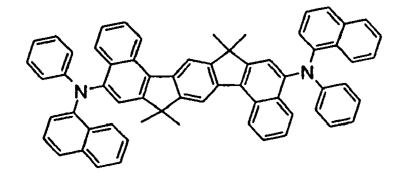 |
| (107) | (108) |
| 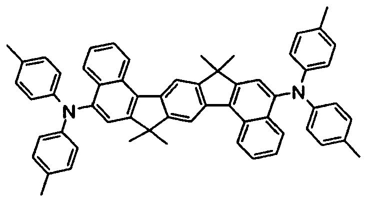 | 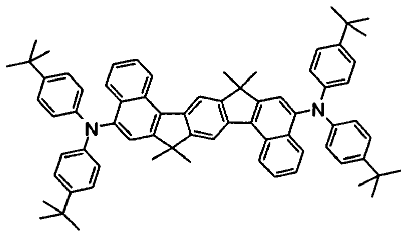 |
| (109) | (110) |
| 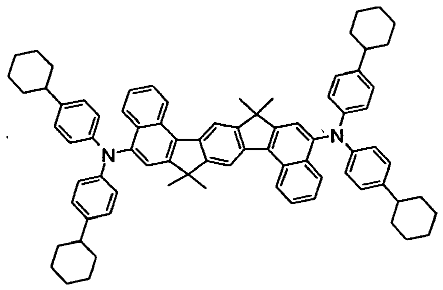 | 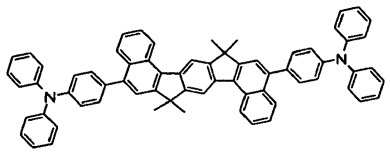 |
| (111) | (112) |
| 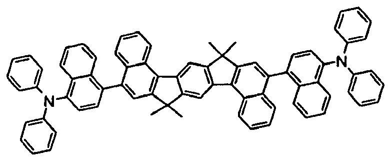 | 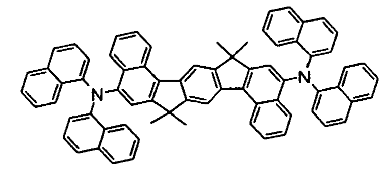 |
| (113) | (114) |
| 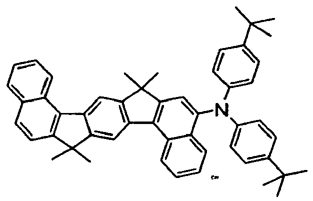 | 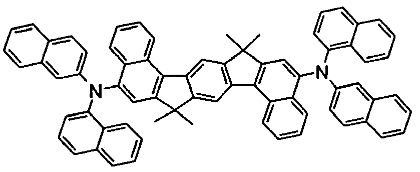 |
| (115) | (116) |
| 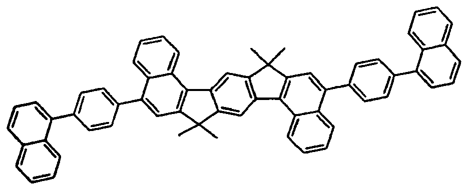 | 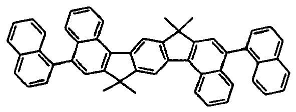 |
| (117) | (118) |
| 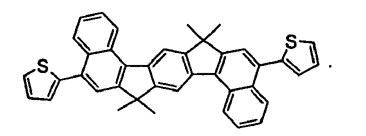 | 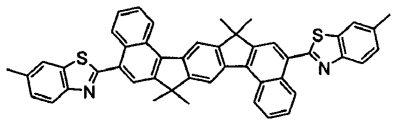 |
| (119) | (120) |
| 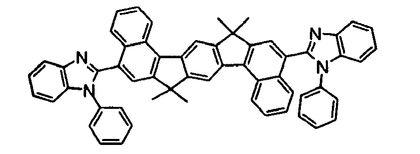 | 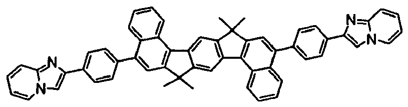 |
| (121) | (122) |
| 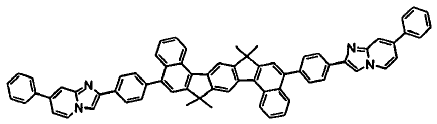 | 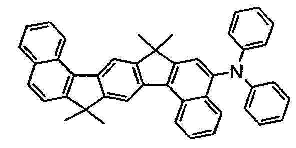 |
| (123) | (124) |
| 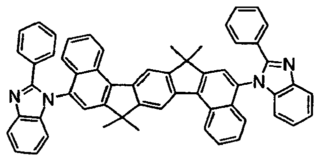 | 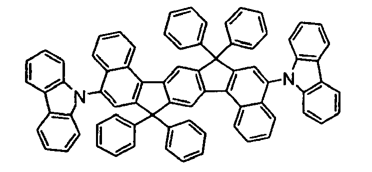 |
| (125) | (126) |
| 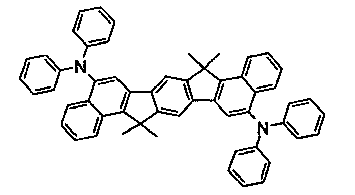 | 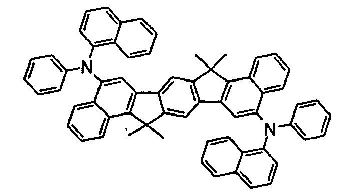 |
| (127) | (128) |
| 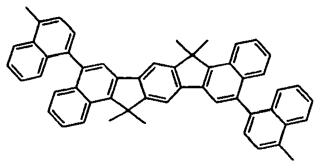 | 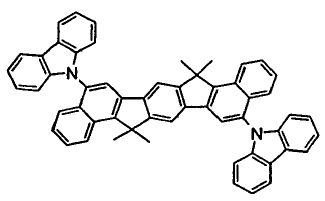 |
| (129) | (130) |
| 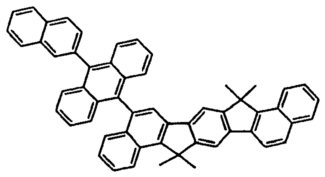 | 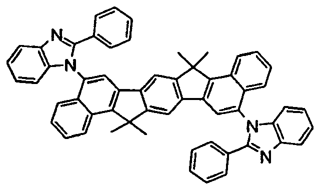 |
| (131) | (132) |

Die oben beschriebenen erfindungsgemäßen Verbindungen, insbesondere Verbindungen, welche mit reaktiven Abgangsgruppen, wie Brom, lod, Boronsäure oder Boronsäureester substituiert sind, können auch als Comonomere zur Erzeugung entsprechender konjugierter, teilkonjugierter oder nicht-konjugierter Polymere, Oligomere oder auch als Kern von Dendrimeren Verwendung finden. Die Polymerisation erfolgt dabei bevorzugt über die Halogenfunktionalität bzw. die Boronsäurefunktionalität.

Weiterer Gegenstand der Erfindung sind somit Polymere, Oligomere oder Dendrimere enthaltend eine oder mehrere Verbindungen gemäß Formel (1) bis (16) bzw. (5a) bis (11a), wobei ein oder mehrere Reste R¹ Bindungen der Verbindung gemäß Formel (1) bis (16) bzw. (5a) bis (11a) zum Polymer oder Dendrimer darstellen. Diese Polymere, Oligomere oder Dendrimere können konjugiert, teilkonjugiert oder nicht-konjugiert sein.

Für die Wiederholeinheiten gemäß Formel (1) bis Formel (16) bzw. (5a) bis (11a) gelten dieselben Bevorzugungen wie oben beschrieben.

Diese Verbindungen werden homopolymerisiert oder mit weiteren Monomeren copolymerisiert. Geeignete und bevorzugte Comonomere sind gewählt aus Fluorenen (z. B. gemäß EP 842208 oder WO 00/22026), Spirobifluorenen (z. B. gemäß EP 707020, EP 894107 oder WO 06/061181), Para-phenylenen (z. B. gemäß WO 92/18552), Carbazolen (z. B. gemäß WO 04/070772 oder WO 04/113468), Thiophenen (z. B. gemäß EP 1028136), Dihydrophenanthrenen (z. B. gemäß WO 05/014689), cis- und trans-Indenofluorenen (z. B. gemäß WO 04/041901 oder WO 04/113412), Ketonen (z. B. gemäß WO 05/040302), Phenanthrenen (z. B. gemäß WO 05/104264 oder der nicht offen gelegten Anmeldung DE 102005037734.3) oder auch mehreren dieser Einheiten. Diese Polymere enthalten üblicherweise noch weitere Einheiten, beispielsweise emittierende (fluoreszierende oder phosphoreszierende) Einheiten, wie z. B. Vinyltriarylamine (z. B. gemäß der nicht offen gelegten Anmeldung DE 102005060473.0) oder phosphoreszierende Metallkomplexe (z. B. gemäß WO 06/003000), und/oder Ladungstransporteinheiten, insbesondere solche basierend auf Triarylaminen.

Die erfindungsgemäßen Verbindungen gemäß Formel (1) bis (16) bzw. (5a) bis (11a) können nach dem Fachmann bekannten Syntheseschritten dargestellt werden. So lassen sich die verschiedenen Grundgerüste beispielsweise durch säurekatalysierte Cyclisierung der entsprechenden tertiären Alkohole darstellen, wie allgemein in Schema 1 für die Verbindungen gemäß Formel (1) gezeigt. Dieses Grundgerüst lässt sich nach Standardmethoden funktionalisieren, beispielsweise durch Friedel-Crafts-Alkylierung oder -Acylierung. Weiterhin lässt sich das Grundgerüst nach Standardmethoden der organischen Chemie bromieren. Die bromierten Verbindungen stellen die Basis für weitere Funktionalisierungen dar. So können sie durch Suzuki-Kupplung mit Arylboronsäuren oder Arylboronsäurederivaten oder mit Organozinnverbindungen gemäß Stille zu erweiterten aromatischen Verbindungen umgesetzt werden. Durch Kupplung mit aromatischen oder aliphatischen Aminen gemäß Hartwig-Buchwald erhält man die entsprechenden Amine. Weiterhin können die bromierten Derivate via Lithiierung und Reaktion mit Elektrophilen wie Benzonitril und anschließender saurer Hydrolyse zu Ketonen oder mit Chlordiphenylphosphinen und anschließender Oxidation zu Phosphinoxiden umgesetzt werden.

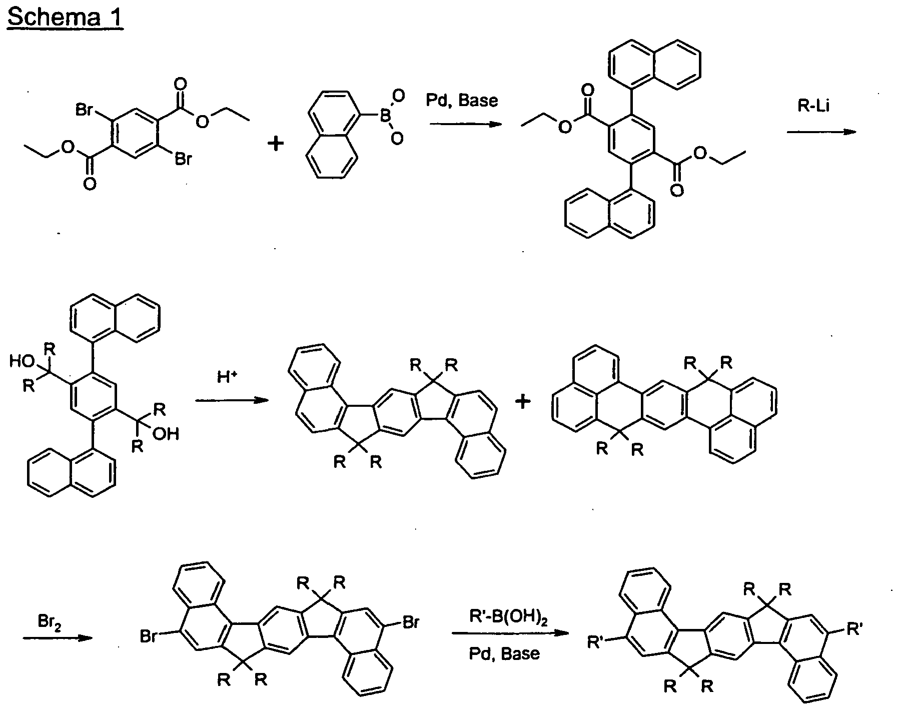

Das oben beschriebene Verfahren führt völlig analog zu Verbindungen gemäß Formel (3) bzw. Formel (4), wenn im ersten Kupplungsschritt statt 1-Naphthylboronsäure 2-Naphthylboronsäure eingesetzt wird.

Weiterhin können die bromierten Verbindungen entweder direkt oder nach Überführung in ein Boronsäurederivat als Monomere für Polymere, Oligomere oder Dendrimere eingesetzt werden.

Bei der Synthese entstehen, je nach Synthesebedingungen, sowohl die 5-Ring/5-Ring-Derivate wie auch die 6-Ring/6-Ring-Derivate, die 5-Ring/6-Ring-Derivate oder Mischungen dieser Verbindungen. Diese können entweder getrennt und als Reinverbindungen weiterverarbeitet werden, oder sie können auch als Mischung eingesetzt werden.

Ein weiterer Gegenstand der vorliegenden Erfindung ist ein Verfahren zur Synthese von Verbindungen gemäß den Formeln (1) bis (4), enthaltend die folgenden Schritte:
a) Kupplung eines funktionalisierten Naphthalins mit einem tetrafunktionalisierten Benzolderivat;
b) gegegebenenfalls weitere Funktionalisierung der funktionellen Gruppen am Benzolderivat;
c) Ringschluss unter Säurekatalyse; und
d) Funktionalisierung des durch die Schritte a) bis c) erhaltenen Grundkörpers durch Bromierung, gefolgt von Hartwig-Buchwald-Kupplung mit einem aromatischen Amin oder Suzuki-Kupplung mit einer Arylboronsäure bzw. einem Arylboronsäurederivat.

Dabei ist die funktionelle Gruppe am Naphthalin bevorzugt eine Boronsäuregruppe bzw. ein Boronsäurederivat. Die zwei funktionellen Gruppen für die Kupplung am Benzolderivat sind bevorzugt Chlor, Brom,oder lod, besonders bevorzugt Brom. Die weiteren zwei funktionellen Gruppen am Benzolderivat sind bevorzugt zwei Estergruppen der Formel C(=O)-O-R, wobei R für eine Alkylgruppe mit 1 bis 20 C-Atomen steht. Die weitere Funktionalisierung im Schritt b) kann beispielsweise durch Addition einer Organolithium-Verbindung erfolgen, wodurch ein tertiärer Alkohol erhalten wird.

Die Verbindungen gemäß Formel (1) bis Formel (16) bzw. (5a) bis (11a) eignen sich für den Einsatz in organischen Elektrolumineszenzvorrichtungen (OLEDs, PLEDs). Abhängig von der Substitution werden die Verbindungen in unterschiedlichen Funktionen und Schichten in der OLED eingesetzt.

Ein weiterer Gegenstand der Erfindung ist daher die Verwendung von Verbindungen gemäß Formel (1) bis (16) bzw. (5a) bis (11a) und insbesondere der oben aufgeführten bevorzugten Ausführungsformen in organischen elektronischen Vorrichtungen, insbesondere in organischen Elektrolumineszenzvorrichtungen.

Nochmals ein weiterer Gegenstand der Erfindung sind organische elektronische Vorrichtungen, enthalten mindestens eine Verbindung ausgewählt aus Verbindungen gemäß Formel (1) bis (16) bzw. (5a) bis (11a), insbesondere organische Elektrolumineszenzvorrichtungen, enthaltend Anode, Kathode und mindestens eine emittierende Schicht, dadurch gekennzeichnet, dass mindestens eine Schicht mindestens eine Verbindung ausgewählt aus Verbindungen gemäß Formel (1) bis (16) bzw. (5a) bis (11a) enthält, inbesondere die oben aufgeführten bevorzugten Ausführungsformen.

Außer Kathode, Anode und der emittierenden Schicht kann die organische Elektrolumineszenzvorrichtung noch weitere Schichten enthalten. Diese können beispielsweise ausgewählt sein aus: Lochinjektionsschicht, Lochtransportschicht, Elektronentransportschicht, Elektroneninjektionsschicht und/oder Charge-Generation Layer (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer). Es sei aber darauf hingewiesen, dass nicht notwendigerweise jede dieser Schichten vorhanden sein muss.

In einer weiteren bevorzugten Ausführungsform der Erfindung enthält die organische Elektrolumineszenzvorrichtung mehrere emittierende Schichten, wobei mindestens eine Schicht mindestens eine Verbindung gemäß Formel (1) bis (16) bzw. (5a) bis (11 a) enthält. Besonders bevorzugt weisen diese Emissionsschichten insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die gelbes, orange oder rotes Licht emittieren. Insbesondere bevorzugt sind Dreischichtsysteme, wobei mindestens eine dieser Schichten mindestens eine Verbindung gemäß Formel (1) bis (16) bzw. (5a) bis (11a) enthält und wobei die drei Schichten blaue, grüne und orange oder rote Emission zeigen (für den prinzipiellen Aufbau siehe z. B. WO 05/011013). Ebenso eignen sich für weiße Emission Emitter, welche breitbandige Emissionsbanden aufweisen und dadurch weiße Emission zeigen.

In einer Ausführungsform der Erfindung werden die Verbindungen gemäß Formel (1) bis (16) bzw. (5a) bis (11a) als Host für einen fluoreszierenden Dotanden eingesetzt. In diesem Fall sind bevorzugt ein oder mehrere Substituenten R¹ aus einfachen oder kondensierten Aryl- oder Heteroarylgruppen gewählt, insbesondere Phenyl, o-, m- oder p-Biphenyl, 1- oder 2-Naphthyl, Anthryl, insbesondere Phenylanthryl oder 1- oder 2-Naphthylanthryl, 2-Fluorenyl und 2-Spirobifluorenyl, welche jeweils durch einen oder mehrere Reste R² substituiert sein können. Dies gilt insbesondere für die Reste R¹ an den Strukturen gemäß Formel (5a) bis (11a).

Unter einem Hostmaterial wird in einem System aus Host und Dotand diejenige Komponente verstanden, die in dem System im höheren Anteil vorliegt. Bei einem System aus einem Host und mehreren Dotanden wird als Host diejenige Komponente verstanden, deren Anteil der höchste in der Mischung ist.

Der Anteil des erfindungsgemäßen Hostmaterials in der emittierenden Schicht beträgt zwischen 50.0 und 99.9 Gew.-%, bevorzugt zwischen 80.0 und 99.5 Gew.-%, besonders bevorzugt zwischen 90.0 und 99.0 Gew.-%. Entsprechend beträgt der Anteil des Dotanden zwischen 0.1 und 50.0 Gew.-%, bevorzugt zwischen 0.5 und 20.0 Gew.-%, besonders bevorzugt zwischen 1.0 und 10.0 Gew.-%.

Bevorzugte Dotanden in fluoreszierenden Vorrichtungen sind gewählt aus der Klasse der Monostyrylamine, der Distyrylamine, der Tristyrylamine, der Tetrastyrylamine und der Arylamine. Unter einem Monostyrylamin wird eine Verbindung verstanden, die eine Styrylgruppe und mindestens ein Amin enthält, welches bevorzugt aromatisch ist. Unter einem Distyrylamin wird eine Verbindung verstanden, die zwei Styrylgruppen und mindestens ein Amin enthält, welches bevorzugt aromatisch ist. Unter einem Tristyrylamin wird eine Verbindung verstanden, die drei Styrylgruppen und mindestens ein Amin enthält, welches bevorzugt aromatisch ist. Unter einem Tetrastyrylamin wird eine Verbindung verstanden, die vier Styrylgruppen und mindestens ein Amin enthält, welches bevorzugt aromatisch ist. Unter einem Arylamin bzw. einem aromatischen Amin im Sinne dieser Erfindung wird eine Verbindung verstanden, die drei aromatische oder heteroaromatische Ringsysteme direkt an den Stickstoff gebunden enthält, davon bevorzugt mindestens ein kondensiertes Ringsystem mit mindestens 14 aromatischen Ringatomen, wenn im System keine Styrylgruppe vorhanden ist. Die Styrylgruppen sind besonders bevorzugt Stilbene, die auch an der Doppelbindung oder an den Aromaten weiter substituiert sein können. Beispiele für derartige Dotanden sind substituierte oder unsubstituierte Tristilbenamine oder weitere Dotanden, die beispielsweise in WO 06/000388, WO 06/058737, WO 06/000389 und in den nicht offen gelegten Patentanmeldungen DE 102005058543.4 und DE 102006015183.6 beschrieben sind. Außerdem sind Verbindungen gemäß WO 06/122630 bevorzugt, ebenso die unten aufgeführten erfindungsgemäßen Dotanden der Formel (1) bis (16) bzw. (5a) bis (11a).

In einer weiteren Ausführungsform der Erfindung werden die Verbindungen gemäß Formel (1) bis (16) bzw. (5a) bis (11a) als Matrix für phosphoreszierende Dotanden eingesetzt. In diesem Fall enthalten bevorzugt ein oder mehrere Substituenten R¹ und/oder Brücken X mindestens eine Gruppe C=O, P(=O) und/oder SO₂. Besonders bevorzugt sind diese Gruppen direkt an die erfindungsgemäße zentrale Einheit gebunden und enthalten weiterhin besonders bevorzugt noch einen bzw. im Fall des Phosphinoxids zwei weitere aromatische Substituenten. Dies gilt insbesondere für die Reste R¹ an den Strukturen gemäß Formel (5a) bis (11a).

In phosphoreszierenden Vorrichtungen ist der Dotand bevorzugt ausgewählt aus der Klasse der Metallkomplexe, enthaltend mindestens ein Element der Ordnungszahl größer 20, bevorzugt größer 38 und kleiner 84, besonders bevorzugt größer 56 und kleiner 80. Bevorzugt werden Metallkomplexe verwendet, die Kupfer, Molybdän, Wolfram, Rhenium, Ruthenium, Osmium, Rhodium, Iridium, Palladium, Platin, Silber, Gold oder Europium enthalten, insbesondere Iridium oder Platin. Generell eignen sich hierfür phosphoreszierende Materialien, wie sie gemäß dem Stand der Technik verwendet werden.

In nochmals einer weiteren Ausführungsform der Erfindung werden die Verbindungen gemäß Formel (1) bis (16) bzw. (5a) bis (11 a) als emittierende Materialien eingesetzt. Die Verbindungen sind insbesondere dann als emittierende Verbindungen geeignet, wenn mindestens ein Substituent R¹ mindestens eine Vinylaryl-Einheit, mindestens eine Vinylarylamin-Einheit und/oder mindestens eine Diarylamino-Einheit N(Ar)₂ enthält. Dies gilt insbesondere für die Reste R¹ an den Strukturen gemäß Formel (5a) bis (11a). Bevorzugt sind die beiden Reste R¹ an den Strukturen gemäß Formel (5a) bis (11 a) gleich gewählt.

Der Anteil der emittierenden Verbindung gemäß Formel (1) bis (16) bzw. (5a) bis (11a) liegt in der Mischung der emittierenden Schicht zwischen 0.1 und 50.0 Gew.-%, bevorzugt zwischen 0.5 und 20.0 Gew.-%, besonders bevorzugt zwischen 1.0 und 10.0 Gew.-%. Entsprechend beträgt der Anteil des Hostmaterials zwischen 50.0 und 99.9 Gew.-%, bevorzugt zwischen 80.0 und 99.5 Gew.-%, besonders bevorzugt zwischen 90.0 und 99.0 Gew.-%.

Als Hostmaterialien kommen hierfür verschiedene Stoffklassen in Frage. Bevorzugte Hostmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 04/081017), der lochleitenden Verbindungen (z. B. gemäß WO 04/058911), der elektronenleitenden Verbindungen, insbesondere Ketone, Phosphinoxide, Sulfoxide, etc. (z. B. gemäß WO 05/084081 und WO 05/084082), der Atropisomere (z. B. gemäß WO 06/048268) oder der Boronsäurederivate (z. B. gemäß WO 06/117052). Weiterhin kommen als Hostmaterialien auch die oben beschriebenen erfindungsgemäßen Verbindungen gemäß den Formeln (1) bis (16) bzw. (5a) bis (11a) in Frage. Besonders bevorzugte Hostmaterialien sind außer den erfindungsgemäßen Verbindungen ausgewählt aus den Klassen der Oligoarylene, enthaltend Naphthalin, Anthracen und/oder Pyren oder Atropisomere dieser Verbindungen, der Oligoarylenvinylene, der Ketone, der Phosphinoxide und der Sulfoxide. Ganz besonders bevorzugte Host-materialien sind außer den erfindungsgemäßen Verbindungen ausgewählt aus den Klassen der Oligoarylene, enthaltend Anthracen und/oder Pyren oder Atropisomere dieser Verbindungen, der Phosphinoxide und der Sulfoxide, insbesondere Anthracen-Derivate, welche mit zwei aromatischen Gruppen substituiert sind, die gleich oder verschieden sein können. Unter einem Oligoarylen im Sinne dieser Erfindung soll eine Verbindung verstanden werden, in der mindestens drei Aryl- bzw. Arylengruppen aneinander gebunden sind.

In nochmals einer weiteren Ausführungsform der Erfindung werden die Verbindungen gemäß Formel (1) bis (16) bzw. (5a) bis (11a) als Lochtransportmaterial bzw. als Lochinjektionsmaterial eingesetzt. Die Verbindungen sind dann bevorzugt mit mindestens einer Gruppe N(Ar)₂ substituiert, bevorzugt mit mindestens zwei Gruppen N(Ar)₂. Die Gruppen N(Ar)₂ sind bevorzugt ausgewählt aus den oben beschriebenen Formeln (17) oder (18). Dies gilt insbesondere für die Reste R¹ an den Strukturen gemäß Formel (5a) bis (11a). Die Verbindung wird bevorzugt in einer Lochtransport- bzw. in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen einer Lochinjektionsschicht und einer Emissionsschicht liegt. Wenn erfindungsgemäßen Verbindungen als Lochtransport- bzw. als Lochinjektionsmaterial verwendet werden, kann es bevorzugt sein, wenn sie mit Elektronenakzeptor-Verbindungen dotiert werden, beispielsweise mit F₄-TCNQ oder mit Verbindungen, wie in EP 1476881 oder EP 1596445 beschrieben.

In nochmals einer weiteren Ausführungsform der Erfindung werden die Verbindungen gemäß Formel (1) bis (16) bzw. (5a) bis (11a) als Elektronentransportmaterial eingesetzt. Hier ist es bevorzugt, wenn ein oder mehrere Substituenten R¹ mindestens eine Einheit C=O, P(=O) und/oder SO₂ enthalten. Besonders bevorzugt sind diese Gruppen direkt an die erfindungsgemäße zentrale Einheit gebunden und enthalten weiterhin besonders bevorzugt noch einen bzw. im Fall des Phosphinoxids zwei weitere aromatische Substituenten. Dies gilt insbesondere für die Reste R¹ an den Strukturen gemäß Formel (5a) bis (11a). Weiterhin kann es bevorzugt sein, wenn die Verbindung mit Elektronendonator-Verbindungen dotiert ist.

Auch in Polymeren können Wiederholeinheiten gemäß Formel (1) bis (16) bzw. (5a) bis (11a) entweder als Polymergrundgerüst (Backbone), als emittierende Einheit, als lochtransportierende Einheit und/oder als elektronentransportierende Einheit eingesetzt werden. Dabei entsprechen die bevorzugten Substitutionsmuster den oben beschriebenen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig. Hohe Löslichkeit lässt sich durch geeignete Substitution der Verbindungen erreichen.

Die erfindungsgemäßen Verbindungen weisen bei Verwendung in organischen Elektrolumineszenzvorrichtungen folgende überraschende Vorteile gegenüber dem Stand der Technik auf:
1. Die erfindungsgemäßen Verbindungen weisen ein tieferes LUMO (niedrigstes unbesetztes Molekülorbital) auf als Verbindungen, welche üblicherweise gemäß dem Stand der Technik verwendet werden, und sind dadurch leichter reduzierbar. Daraus resultiert eine verbesserte Elektroneninjektion und damit eine Verringerung der Betriebsspannung.
2. Die erfindungsgemäßen Verbindungen weisen einen geringen Bandabstand auf und sind damit als bipolare Strukturen zu betrachten, welche sowohl gut reduziert, wie auch gut oxidiert werden können. Dadurch eignen sie sich gleichermaßen zur Loch- wie auch zur Elektroneninjektion. Diese Verbindungen sind damit ein Schritt in Richtung von emittierenden Schichten, welche nur eine Reinsubstanz enthalten. Dies stellt eine technische Vereinfachung bei der Device-Herstellung dar.
3. Die erfindungsgemäßen Verbindungen führen bei Verwendung in organischen Elektrolumineszenzvorrichtungen zu gegenüber dem Stand der Technik deutlich verbesserten Lebensdauern.
4. Die erfindungsgemäßen Verbindungen weisen eine sehr hohe thermische Stabilität auf, was sowohl die problemlose Massensublimation zur Reinigung der Verbindungen wie auch das zersetzungsfreie thermische Aufdampfen der Verbindungen bei der Herstellung der organischen Elektrolumineszenzvorrichtungen ermöglicht.

Im vorliegenden Anmeldetext wird auf die Verwendung der erfindungsgemäßen Verbindungen in Bezug auf OLEDs und PLEDs und die entsprechenden Displays abgezielt. Trotz dieser Beschränkung der Beschreibung ist es für den Fachmann ohne weiteres efinderisches Zutun möglich, die erfindungsgemäßen Verbindungen auch für weitere Verwendungen in anderen elektronischen Vorrichtungen einzusetzen, z. B. für organische Feld-Effekt-Transistoren (O-FETs), organische Dünnfilmtransistoren (O-TFTs), organische lichtemittierende Transistoren (O-LETs), organische integrierte Schaltungen (O-ICs), organische Solarzellen (O-SCs), organische Feld-Quench-Devices (O-FQDs), lichtemittierende elektrochemische Zellen (LECs), organische Laserdioden (O-Laser) oder organische Photorezeptoren.

Die Verwendung der erfindungsgemäßen Verbindungen in den entsprechenden Vorrichtungen ebenso wie diese Vorrichtungen selbst sind ebenfalls ein Gegenstand der vorliegenden Erfindung.

Die Erfindung wird durch die nachfolgenden Beispiele näher erläutert, ohne sie dadurch einschränken zu wollen.

### Beispiele:

Die nachfolgenden Synthesen wurden, sofern nicht anders angegeben, unter einer Schutzgasatmosphäre durchgeführt. Die Edukte wurden von den Firmen ALDRICH bzw. ABCR bezogen.

### Beispiel 1: Synthese von 3,8-Bis-(N,N-Diphenylamino)-1,2,6,7-dibenzo-6,6,12,12-tetramethyl-6,12-dihydro-indeno[1,2-b]fluoren

### a) 2,5-Dinaphth-1-yl-terephthalsäurediethylester

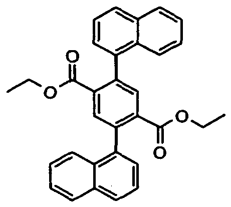

193.3 g (0.82 mol) Naphthylboronsäure, 180.4 g (474 mmol) Dibromterephthalsäure-diethylester und 315.9 g (2.29 mol) Kaliumcarbonat werden im Gemisch von 850 mL Toluol und 850 mL Wasser vorgelegt und für 30 min. mit N₂ gesättigt. Nach Zugabe von 1.36 g (1.18 mmol) Pd(PPh₃)₄ wird die Mischung für 4 h zum Sieden erhitzt. Nach Abkühlen auf RT und Zugabe von 400 mL EtOH wird auf Raumtemperatur abgekühlt, 1 h gerührt, der Niederschlag abgesaugt, mit Wasser, EtOH und Heptan gewaschen und bei 80 °C im Vakuum getrocknet. Die Ausbeute an farblosem Feststoff beträgt 160.7g (71 %).

### b) 2-[4-(1-Hydroxy-1-methylethyl)-2,5-dinaphth-1-yl-phenyl]propan-2-ol

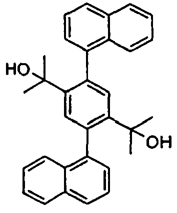

64.6 g (136.4 mmol) 2,5-Dinaphth-1-yl-terephthalsäurediethylester werden in 600 mL THF vorgelegt, auf -70 °C gekühlt und 400 mL (600 mmol) 1.6 M Methyllithium-Lösung innerhalb 60 min bei -70 °C zugetropft. Nach 2 h bei -70 °C werden zunächst 30 mL Eiswasser, dann 60 mL 50 % Essigsäure zugetropft, das Reaktionsgemisch extraktiv mit Essigsäureethylester/Wasser aufgearbeitet, die organische Phase über Na₂SO₄ getrocknet und im Vakuum vom Lösungsmittel befreit. Man erhält 60.3 g (99 %) eines farblosen Feststoffes.

### c) 1,2,6,7-Dibenzo-6,6,12,12-tetramethyl-6,12-dihydro-indeno[1,2-b]fluoren

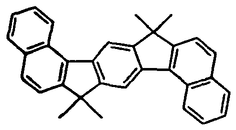

34.12 g (76 mmol) 2-[4-(1-Hydroxy-1-methylethyl)-2,5-dinaphth-1-yl-phenyl]-propan-2-ol werden in 600 mL Toluol vorgelegt und nach Zugabe von 1 mL konz. H₂SO₄ am Wasserabscheider bis zur Beendigung der Wasserabgabe zum Sieden erhitzt. Nach Abkühlen auf RT wird das ausgefallene Reaktionsprodukt (nach ¹H-NMR ausschließlich 5,5-Ring) abgesaugt und aus NMP umkristallisiert. Man erhält ein gelbliches Pulver mit einer Reinheit > 99.5 % in einer Ausbeute von 32 g (78 %).

### d) 3,8-Dibrom-1,2,6,7-dibenzo-6,6,12,12-tetramethyl-6,12-dihydro-indeno[1,2-b]fluoren

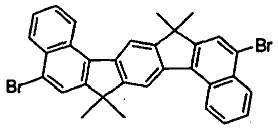

17.5 g (43 mmol) 1,2,6,7-Dibenzo-6,6,12,12-tetramethyl-6,12-dihydro-indeno[1,2-b]fluoren werden unter Lichtausschluss in 500 mL Dichlormethan vorgelegt, auf 5 °C abgekühlt, 4.7 mL (86 mmol) Brom in 40 mL Dichlormethan innerhalb 15 min zugetropft und bei 5 °C für 6 h gerührt. Die Reaktion wird per HPLC-Analyse von Reaktionsaliquots kontrolliert. Nach vollständigem Umsatz wird die Reaktion durch Zugabe von 20 mL EtOH gestoppt, abgesaugt, mehrfach mit EtOH gewaschen und anschließend zweimal aus NMP umkristallisiert. Man erhält 21.3 g (88%) eines hellgelben Feststoffes, der laut HPLC-Analyse einen Gehalt von > 99.8 % aufweist.

### e) 3,8-Bis-(N,N-Diphenylamino)-1,2,6,7-dibenzo-6,6,12,12-tetramethyl-6,12-dihydro-indeno[1,2-b]fluoren

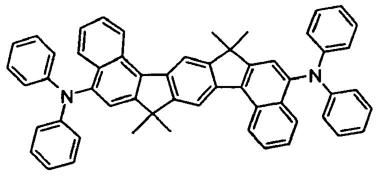

40 g (70 mmol) 3,8-Dibrom-1,2,6,7-dibenzo-6,6,12,12-tetramethyl-6,12-dihydro-indeno[1,2-b]fluoren und 31 g (184 mmol) Diphenylamin werden in 750 mL wasserfreiem Toluol suspendiert, anschließend 600 mg (3 mmol) Tri-tert-butylphosphin sowie 340 mg (1.5 mmol) Pd(OAc)₂ und 20 g (211 mmol) NaO^{t}Bu zugegeben und das Reaktionsgemisch für 4 h zum Sieden erhitzt. Nach beendeter Reaktion werden 400 mL Wasser zugegeben, der Feststoff abgesaugt, mit EtOH gewaschen und getrocknet. Vierfache Umkristallisation aus NMP, einmaliges Auskochen in Ethanol und anschließende zweifache Sublimation (340 °C, 2 x 10⁻⁵ mbar) liefern 41.7 g (80 %) eines hellgelben Feststoffes mit einer durch HPLC bestimmten Reinheit von > 99.9 %.

In Analogie zum oben beschriebenen Verfahren werden folgende Verbindungen hergestellt (Ausbeuten nach Sublimation bei einer Reinheit von > 99.9 %) (Beispiele 2 bis 9).

| Bsp. | Struktur | Ausbeute (%) |
|---|---|---|
| 2 | 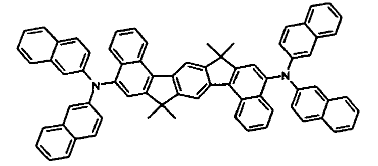 | 67 |
| 3 | 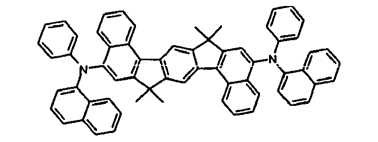 | 73 |
| 4 | 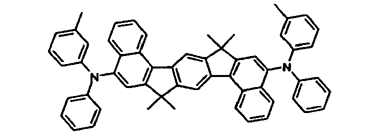 | 84 |
| 5 | 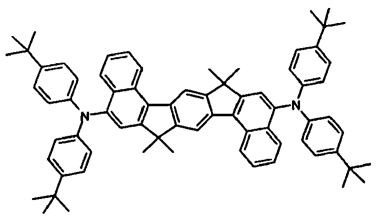 | 64 |
| 6 | 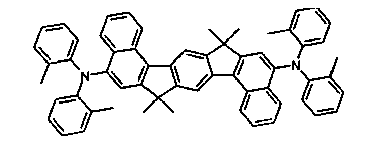 | 55 |
| 7 | 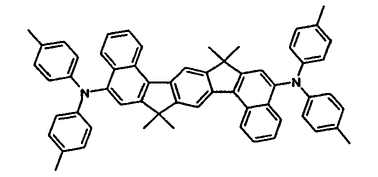 | 88 |
| 8 | 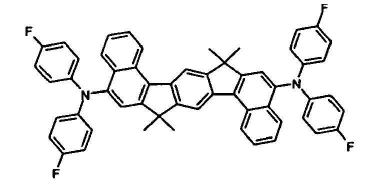 | 77 |
| 9 | 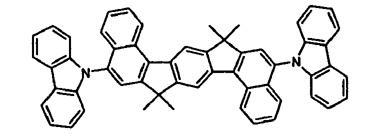 | 54 |

### Beispiel 10: Synthese von 3,8-Bis-(4-triphenylamino)-1,2,6,7-dibenzo-6,6,12,12-tetrarnethyl-6,12-dihydro-indeno[1,2-b]fluoren

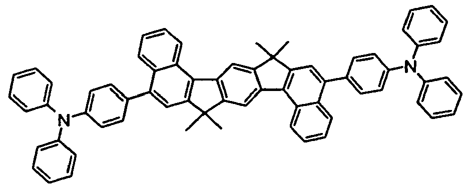

69 g (122 mmol) 3,8-Dibrom-1,2,6,7-dibenzo-6,6,12,12-tetramethyl-6,12-dihydro-indeno[1,2-b]fluoren (aus Beispiel 1d), 115 g (365 mmol) Triphenylamin-4-boronsäureethylenglycolester und 109 g (512 mmol) Trikaliumphosphat werden in einem Gemisch von 450 mL Toluol, 150 mL Dioxan und 600 mL Wasser suspendiert, das Gemisch mit Stickstoff gesättigt, 4.4 g (14.6 mmol) Tris-o-tolylphosphin, gefolgt von 0.5 g (2.4 mmol) Palladiumacetat zugegeben und 12 h zum Sieden erhitzt. Nach Zugabe von 500 mL EtOH wird der Niederschlag abgesaugt, getrocknet und fünfmal aus NMP umkristallisiert. Nach Auskochen in EtOH und zweimaliger Sublimation (370 °C, 2 x 10⁻⁵ mbar) erhält man 70 g (78 mmol) des Diamins mit einer Reinheit > 99.9 % (HPLC).

In Analogie zum oben beschriebenen Verfahren werden folgende Verbindungen hergestellt (Ausbeuten nach Sublimation bei einer Reinheit > 99.9 %) (Beispiele 11 bis 13).

| Bsp. | Struktur | Ausbeute (%) |
|---|---|---|
| 11 | 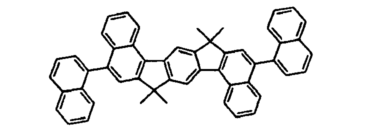 | 54 |
| 12 | 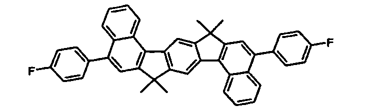 | 77 |
| 13 | 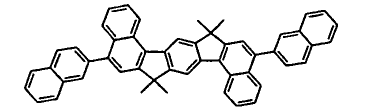 | 65 |

### Beispiel 14: Synthese von 1,2,6,7-Dibenzo-6,6,12,12-tetra-(4-tert-butylphenyl)-6,12-dihydro-indeno[1,2-b]fluoren

### a) {4-[Bis-(4-tert-butyl-phenyl)-hydroxy-methyl]-2,5-dinaphth-1-yl-phenyl}-bis-(4-tert-butyl-phenyl)-methanol

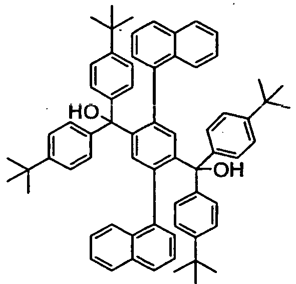

42.5 g (200 mmol) *t*-Butylbrombenzol werden in 200 mL getrocknetem THF vorgelegt, auf -75 °C gekühlt, 100 mL (200 mmol) n-BuLi (2 M in Cyclohexan) innerhalb 30 min zugetropft und weitere 2 h bei -75 °C gerührt. 21.3 g (45 mmol) 2,5-Dinaphth-1-yl-terephthalsäurediethylester (hergestellte gemäß Beispiel 1a) werden in 160 mL trockenem THF gelöst und bei -75 °C innerhalb 30 min zugetropft, 1 h bei -75 °C gerührt und nach Erwärmen auf RT 20 mL 50%ige Essigsäure zugetropft. Das Reaktionsgemisch wird extraktiv mit Essigsäureethylester und Wasser aufgearbeitet, die organische Phase über Na₂SO₄ getrocknet und im Vakuum das Lösungsmittel entfernt. Man erhält das Diol als farblosen Feststoff mit einer Reinheit von > 99 % bestimmt durch ¹H-NMR-Spektroskopie (40.5 g, 98 %).

### b) 1,2,6,7-Dibenzo-6,6,12,12-tetra-(4-tert-butylphenyl)-6,12-dihydro-indeno[1,2-b]fluoren

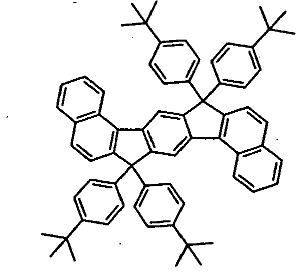

41.4 g (45 mmol) {4-[Bis-(4-tert-butyl-phenyl)-hydroxy-methyl]-2,5-dinaphth-1-yl-phenyl}-bis-(4-tert-butyl-phenyl)-methanol werden in 500 mL konz. Essigsäure suspendiert, 2 mL konz. HCl zugegeben und das Gemisch für 4 h zum Sieden erhitzt. Das Rohprodukt wird abfiltriert und besteht nach ¹H-NMR Analyse aus einem Gemisch von 5,5- und 6,6-Ring verknüpftem Produkt (ca. 3:1). Die Trennung und Reinigung erfolgt durch siebenmalige Umkristallisation aus Dichlorbenzol. Zweifache Zugabe und Abdestillation von Toluol, Auskochen in n-Heptan und abschließende zweifache Sublimation (400 °C, 2 x 10⁻⁵ mbar) liefern das Produkt in Form eines farblosen Feststoffes (16.7 g, 42 %).

Die Verbindung gemäß Beispiel 14 kann analog zu den Beispielen 1 und 10 weiter funktionalisiert werden.

### Beispiel 15: Synthese von 2,7-Bis-N,N-Diphenylamino)-3,4,8,9-dibenzo-6,6,12,12-tetramethyl-6,12-dihydro-indeno[1,2-b]fluoren

### a) 2;7-Bis-(N,N-Diphenylamino)-3,4,8,9-dibenzo-6,6,12,12-tetramethyl-6,12-dihydro-indeno[1,2-b]fluoren

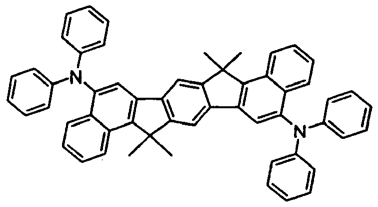

Die Verbindung wird analog zum Beispiel 1 ausgehend von 2-Naphthalinboronsäure anstatt 1-Naphthalinboronsäure hergestellt und durch viermalige Umkristallisation aus Chlorbenzol und abschließende Sublimation (350 °C, 2 x 10⁻⁵ mbar) gereinigt. Die Ausbeute für die Aminierung beträgt 66 % bei einer Reinheit von > 99.9 % (HPLC).

In Analogie zum oben beschriebenen Verfahren werden folgende Verbindungen hergestellt (Ausbeuten nach Sublimation bei einer Reinheit von > 99.9 %) (Beispiele 16 bis 23).

| Bsp. | Struktur | Ausbeute (%) |
|---|---|---|
| 16 | 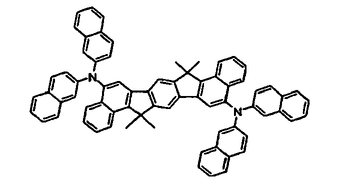 | 88 |
| 17 | 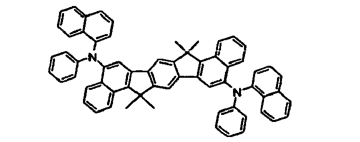 | 71 |
| 18 | 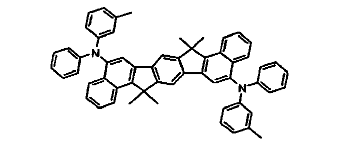 | 82 |
| 19 | 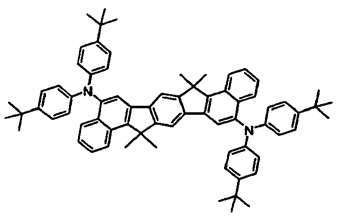 | 66 |
| 20 | 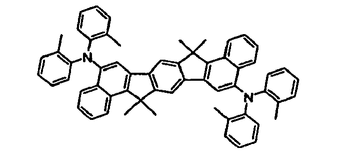 | 49 |
| 21 | 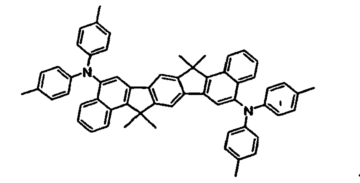 | 86 |
| 22 | 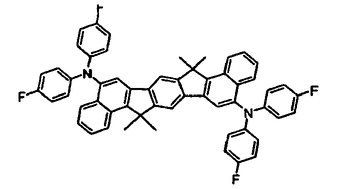 | 75 |
| 23 | 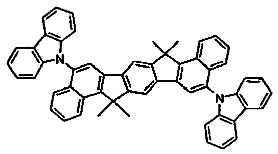 | 59 |

### Beispiel 24: Synthese von 2,7-Bis-(4-triphenylamino)-3,4,8,9-dibenzo-6,6,12,12-tetramethyl-6,12-dihydro-indeno[1,2-b]fluoren

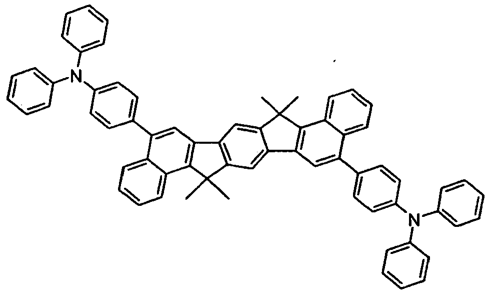

Die Verbindung wird analog zum Beispiel 10 ausgehend von 2-Naphthalinboronsäure anstatt 1-Naphthalinboronsäure hergestellt und durch viermalige Umkristallisation aus NMP und abschließende Sublimation (390 °C, 2 x 10⁻⁵ mbar) gereinigt. Die Ausbeute für die Suzuki Reaktion beträgt 77 % bei einer Reinheit von > 99.9 % (HPLC).

In Analogie zum oben beschriebenen Verfahren werden folgende Verbindungen hergestellt (Ausbeuten nach Sublimation bei einer Reinheit von > 99.9 %) (Beispiele 25 bis 27).

| Bsp. | Struktur | Ausbeute (%) |
|---|---|---|
| 25 | 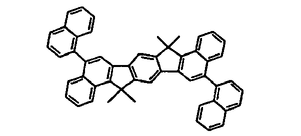 | 58 |
| 26 | 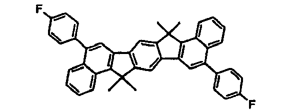 | 82 |
| 27 | 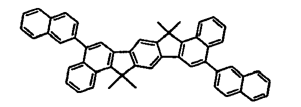 | 78 |

### Beispiel 28: Herstellung der OLEDs

Die Herstellung von OLEDs erfolgt nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wird.

In den folgenden Beispielen 29 bis 47 werden die Ergebnisse verschiedener OLEDs vorgestellt. Glasplatten, die mit strukturiertem ITO (Indium Zinn Oxid) beschichtet sind, bilden die Substrate. Zur verbesserten Prozessierung wird PEDOT (aus Wasser aufgeschleudert; bezogen von H. C. Starck, Goslar, Deutschland; Poly(3,4-ethylendioxy-2,5-thiophen)) direkt auf das Substrat aufgebracht. Die OLEDs bestehen immer aus folgender Schichtenfolge: Substrat / PEDOT 20 nm / Lochinjektionsschicht (HIL1) 20 nm / Lochtransportschicht (HTM1) 20 nm / Emissionsschicht (EML) 30 nm / Elektronentransportschicht (ETM) 20 nm und abschließend eine Kathode. Die Materialien bis auf PEDOT werden in einer Vakuumkammer thermisch aufgedampft. Dabei besteht die EML immer aus einem Matrixmaterial (Host, abgekürzt H) und einem Dotierstoff (Guest oder Dotand, abgekürzt D), der durch Coverdampfung dem Host beigemischt wird. Die Kathode wird durch eine 1 nm dünne LiF-Schicht und eine darauf abgeschiedene 100 nm Al-Schicht gebildet. Die Tabelle 1 zeigt die Strukturen der verwendeten Materialien.

**Tabelle 1: Strukturen der verwendeten Materialien**

| | | |
|---|---|---|
| 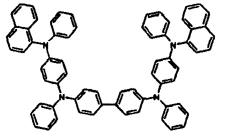 | 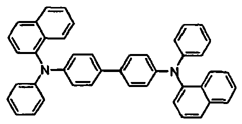 | 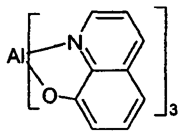 |
| HIL1 | HTM1 | ETM1 |
| 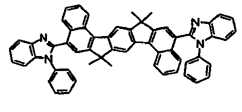 | 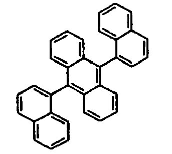 | 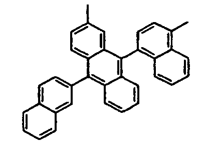 |
| ETM2 | H1 | H2 |
| 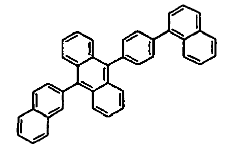 | 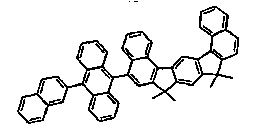 | 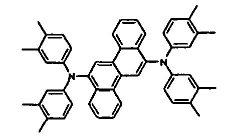 |
| H3 | H4 | D1 |
| 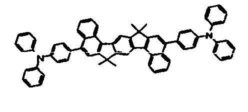 | 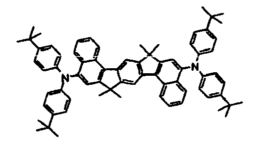 | 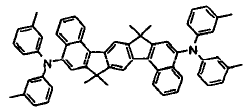 |
| D2 | D3 | D4 |
| 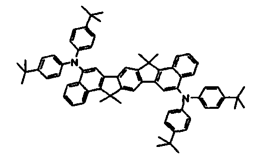 | 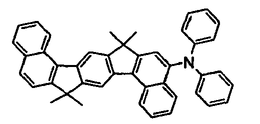 | 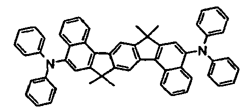 |
| D5 | D6 | D7 |

Dabei ist ETM-1 ein Elektronentransportmaterial gemäß dem Stand der Technik. ETM-2 ist ein erfindungsgemäßes Elektronentransportmaterial. H1, H2 und H3 sind Hostmaterialien gemäß dem Stand der Technik. H4 ist ein erfindungsgemäßes Hostmaterial. D1 ist ein Dotand gemäß dem Stand der Technik. D2, D3, D4, D5, D6 und D7 sind erfindungsgemäße Dotanden.

Die oben beschriebenen OLEDs werden standardmäßig charakterisiert; hierfür werden die Elektrolumineszenzspektren, die Effizienz (gemessen in cd/A), die Leistungseffizienz (gemessen in Im/W) in Abhängigkeit der Helligkeit, berechnet aus Strom-Spannungs-Helligkeit-Kennlinien (IUL-Kennlinien), und die Lebensdauer bestimmt. Als Lebensdauer wird die Zeit definiert, nach der die Anfangshelligkeit von 2000 cd/m² auf die Hälfte gesunken ist.

In Tabelle 2 sind die Schichtaufbauten (Beispiele 29 bis 47) einiger OLEDs aufgeführt und deren Ergebnisse zusammengefasst.

**Tabelle 2: Schichtaufbauten und Ergebnisse der OLEDs**

| **Bsp.** | **EML** | **ETL** | **Max. Effizienz (cd/A)** | **Spannung (V) bei 1000 Cd/m²** | **CIE** | **Lebensdauer (h) bei 2000 cd/m²** |
|---|---|---|---|---|---|---|
| 29 ^{a)} | H3 + 5% D1 | ETM1 | 6.9 | 5.8 | x=0.15 / y=0.20 | 2200 |
| 30 | H4 + 5% D1 | ETM1 | 7.3 | 5.6 | x=0.15 / y=0.19 | 2200 |
| 31 | H3 + 5% D1 | ETM2 | 7.5 | 5.3 | x=0.15 / y=0.19 | 2600 |
| 32 | H1 + 5% D2 | ETM1 | 7.0 | 5.7 | x=0.16 / y=0.20 | 2400 |
| 33 | H3 + 5% D2 | ETM1 | 7.2 | 5.3 | x=0.14 / y=0.19 | 3100 |
| 34 | H4 + 5% D2 | ETM2 | 7.5 | 5.2 | x=0.14/y=0.19 | 2900 |
| 35 | H1 + 5% D3 | ETM1 | 21.4 | 5.1 | x=0.18 / y=0.42 | 6300 |
| 36 | H2 + 5% D3 | ETM1 | 18.4 | 5.2 | x=0.18 / y=0.43 | 7200 |
| 37 | H3 + 5% D3 | ETM1 | 20.2 | 5.3 | x=0.18 y=0.42 | 7500 |
| 38 | H1 + 5% D4 | ETM1 | 12.3 | 5.5 | x=0.15 / y=0.30 | 5200 |
| 39 | H3 + 5% D4 | ETM1 | 12.7 | 5.4 | x=0.14/y=0.29 | 5300 |
| 40 | H3 + 5% D4 | ETM2 | 13.8 | 5.2 | x=0.14 / y=0.29 | 5800 |
| 41 | H1 + 5% D5 | ETM1 | 9.2 | 5.6 | x=0.15/ y=0.26 | 3100 |
| 42 | H2 + 5% D5 | ETM2 | 10.8 | 5.3 | x=0.14 / y=0.26 | 3700 |
| 43 | H3 + 5% D5 | ETM2 | 9.9 | 5.5 | x=0.14 1 y=0.26 | 3800 |
| 44 | H1 + 5% D6 | ETM1 | 6.2 | 5.7 | x=0.15 / y=0.14 | 2000 |
| 45 | H2 + 5% D6 | ETM1 | 6.0 | 5.6 | x=0.14 / y=0.13 | 2200 |
| 46 | H3 + 5% D6 | ETM2 | 6.1 | 5.6 | x=0.14 / y=0.13 | 2400 |
| 47 | H3 + 5% D7 | ETM2 | 12.6 | 5.3 | X=0.14 / y=0.28 | 5900 |

| | | | | | | |
|---|---|---|---|---|---|---|
| ^{a)} Vergleichsexperiment gemäß dem Stand der Technik. | | | | | | |

Wie man aus den Ergebnissen in Tabelle 2 erkennen kann, weisen die erfindungsgemäßen OLED höhere Lebensdauern auf als die OLED gemäß dem Stand der Technik (Beispiel 29).

## Patentansprüche

1. Verbindungen gemäß Formel (1) bis Formel (4), 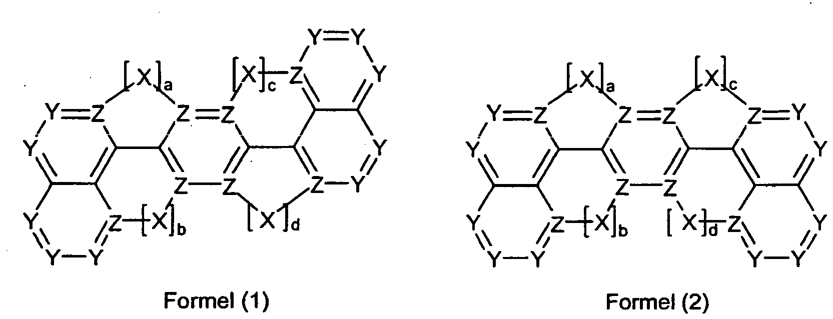 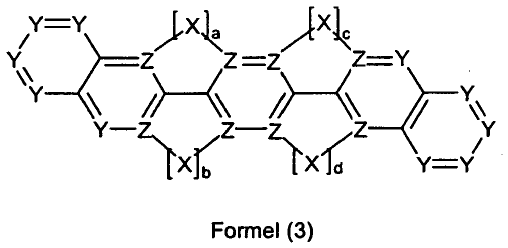 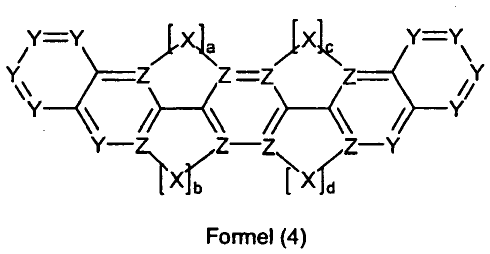 wobei für die Symbole und Indizes gilt:
Y ist bei jedem Auftreten gleich oder verschieden CR¹ oder N;
Z ist gleich C, falls an die Gruppe Z eine Brücke X gebunden ist, und ist gleich Y, falls an die Gruppe Z keine Brücke X gebunden ist;
X ist bei jedem Auftreten gleich oder verschieden eine bivalente Brücke, ausgewählt aus B(R¹), C(R¹)₂, Si(R¹)₂, C=O, C=NR¹, C=C(R¹)₂, O, S, S=O, SO₂, N(R¹), P(R¹) und P(=O)R¹;
R¹ ist bei jedem Auftreten gleich oder verschieden H, F, Cl, Br, I, N(Ar)₂, C(=O)Ar, P(=O)Ar₂, S(=O)Ar, S(=O)₂Ar, CR²=CR²Ar, CN, NO₂, Si(R²)₃, B(OR²)₂, OSO₂R², eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R² substituiert sein kann und wobei eine oder mehrere nicht benachbarte CH₂-Gruppen durch R²C=CR², C=C , Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch F, Cl, Br, I, CN oder NO₂ ersetzt sein können, oder ein aromatisches oder heteroaromatisches Ringsystem mit 5 bis 40 aromatischen Ringatomen, das jeweils durch einen oder mehrere Reste R² substituiert sein kann, oder eine Aryloxy-oder Heteroaryloxygruppe mit 5 bis 40 aromatischen Ringatomen, die durch einen oder mehrere Reste R² substituiert sein kann, oder eine Kombination dieser Systeme; dabei können zwei oder mehrere benachbarte Substituenten R¹ auch miteinander ein mono- oder polycyclisches aliphatisches oder aromatisches Ringsystem bilden;
Ar ist bei jedem Auftreten gleich oder verschieden ein aromatisches oder heteroaromatisches Ringsystem mit 5-30 aromatischen Ringatomen, das mit einem oder mehreren nicht-aromatischen Resten R¹ substituiert sein kann; dabei können auch zwei Reste Ar am selben Stickstoff- oder Phosphoratom durch eine Einfachbindung oder eine Brücke X miteinander verknüpft sein;
R² ist bei jedem Auftreten gleich oder verschieden H oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten R² auch miteinander ein mono- oder polycyclisches aliphatisches oder aromatisches Ringsystem bilden;
a, b, c, d ist bei jedem Auftreten gleich oder verschieden 0 oder 1, mit der Maßgabe, dass a + b = 1 oder 2 und c + d = 1 oder 2 ist, wobei a = 0 bzw. b = 0 bzw. c = 0 bzw. d = 0 jeweils bedeutet, dass die entsprechende Brücke X nicht vorhanden ist;
ausgenommen sind die folgenden Verbindungen:

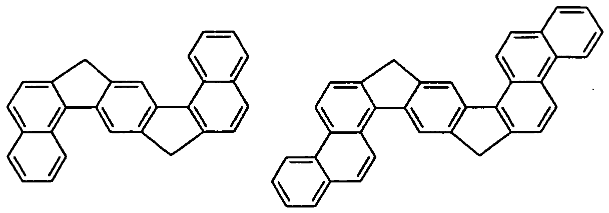

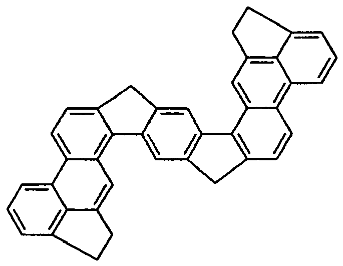

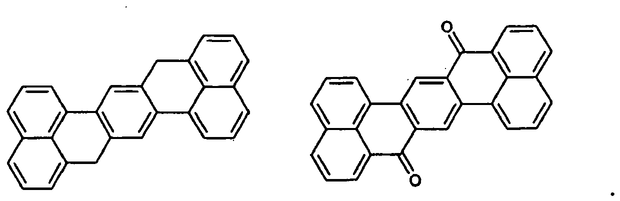

2. Verbindungen nach Anspruch 1, **dadurch gekennzeichnet, dass** für die Indizes a, b, c und d gilt, dass a + b = 1 und c + d = 1 ist.

3. Verbindungen nach Anspruch 1 oder 2 ausgewählt aus den Verbindungen der Formeln (5), (6), (7), (8), (9), (10), (11), (12), (13), (14), (15) oder (16), 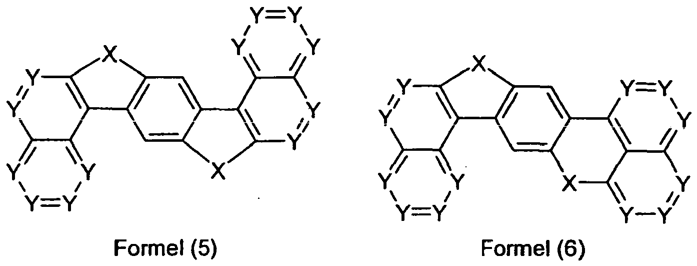 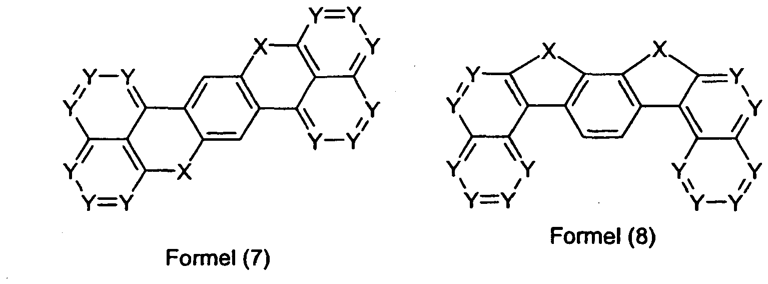 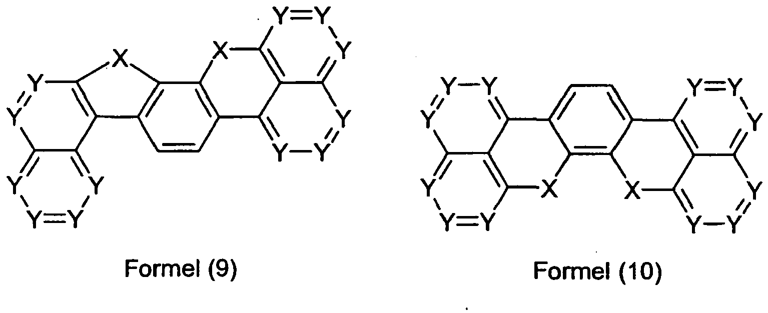 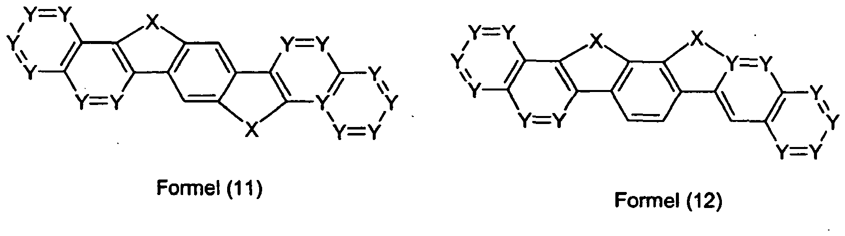 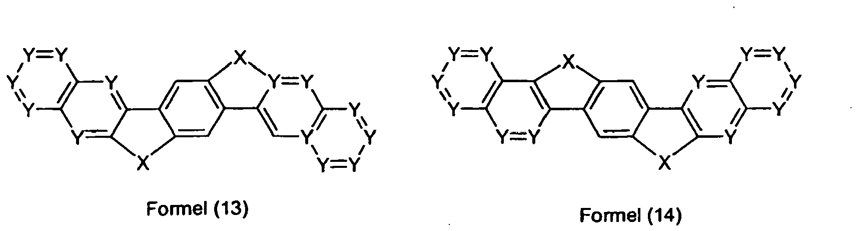 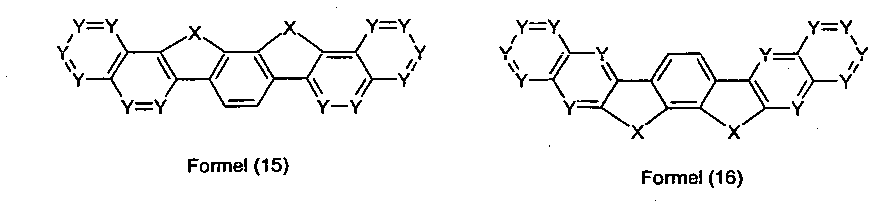 wobei die Symbole X und Y dieselbe Bedeutung haben, wie in Anspruch 1 beschrieben.

4. Verbindungen nach einem oder mehreren der Ansprüche 1 bis 3, **dadurch gekennzeichnet, dass** das Symbol Y insgesamt 0, 1, 2, 3 oder 4 Mal für Stickstoff steht, wobei die anderen Symbole Y für CR¹ stehen.

5. Verbindungen nach einem oder mehreren der Ansprüche 1 bis 4, ausgewählt aus den Formeln (5a), (6a), (7a), (8a), (9a), (10a) oder (11a), 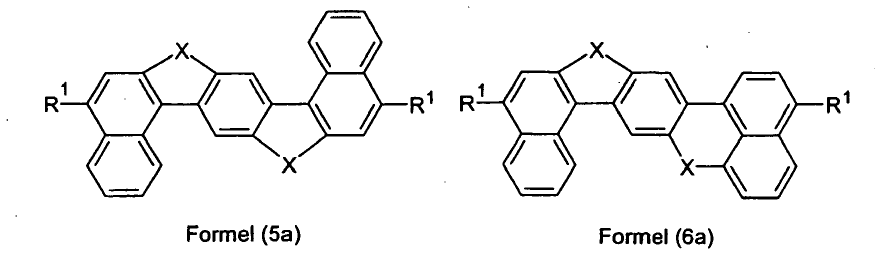 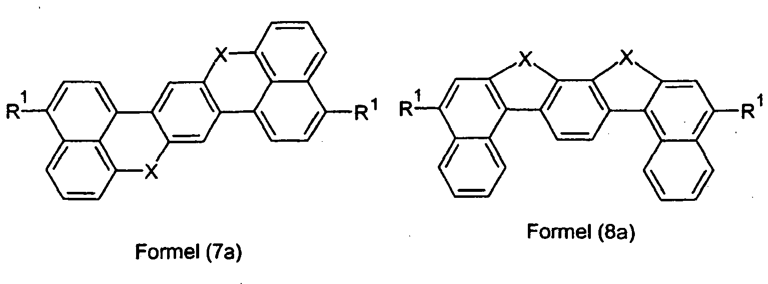 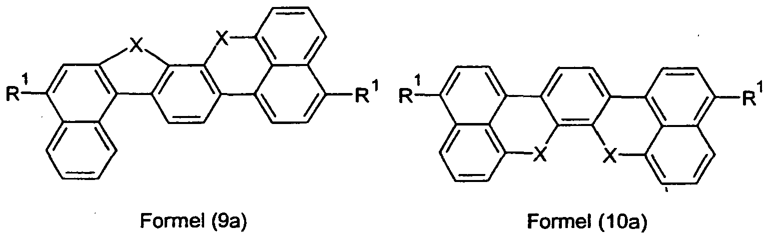 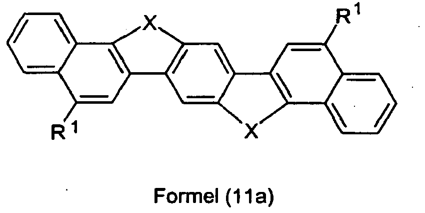 wobei die Symbole X und R¹ dieselbe Bedeutung haben, wie in Anspruch 1 beschrieben.

6. Verbindungen nach einem oder mehreren der Ansprüche 1 bis 5, **dadurch gekennzeichnet, dass** das Symbol R¹ gleich oder verschieden bei jedem Auftreten für H, F, Br, C(=O)Ar, P(=O)Ar₂, CR²=CR²Ar, eine geradkettige Alkylgruppe mit 1 bis 5 C-Atomen oder verzweigte Alkylgruppe mit 3 bis 5 C-Atomen, wobei eine oder mehrere nicht benachbarte CH₂-Gruppen durch -R²C=CR²-, -C=C- oder -O- ersetzt sein können und wobei ein oder mehrere H-Atome durch F ersetzt sein können, oder eine Arylgruppe mit 6 bis 16 C-Atomen oder Heteroarylgruppe mit 2 bis 16 C-Atomen oder eine Spirobifluorengruppe, die jeweils mit einem oder mehreren Resten R² substituiert sein können, oder eine Kombination aus zwei oder drei dieser Systeme steht, und/oder dass mindestens ein Symbol R¹ für eine Gruppe N(Ar)₂ der Formel (17) oder (18) steht, 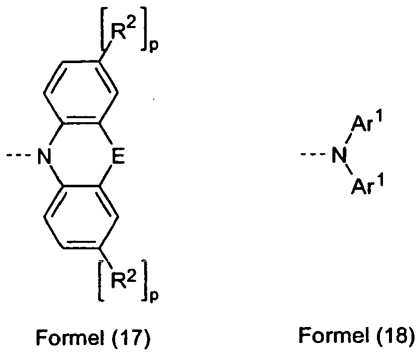 wobei R² die in Anspruch 1 aufgeführte Bedeutung hat und weiterhin gilt:
E steht für eine Einfachbindung, O, S, N(R²) oder C(R²)₂;
Ar¹ ist gleich oder verschieden bei jedem Auftreten eine Aryl- oder Heteroarylgruppe mit 5 bis 20 aromatischen Ringatomen oder eine Triarylamingruppe mit 15 bis 30 aromatischen Ringatomen, welche jeweils mit einem oder mehreren Resten R² substituiert sein kann, bevorzugt eine Aryl- oder Heteroarylgruppe mit 6 bis 14 aromatischen Ringatomen oder eine Triarylamingruppe mit 18 bis 22 aromatischen Ringatomen, welche jeweils mit einem oder mehreren Resten R² substituiert sein kann;
p ist bei jedem Auftreten gleich oder verschieden 0 oder 1.

7. Verbindungen nach einem oder mehreren der Ansprüche 1 bis 6, **dadurch gekennzeichnet, dass** das Symbol X bei jedem Auftreten gleich oder verschieden eine bivalente Brücke ist, ausgewählt aus C(R¹)₂, C=O, C=NR¹, O, S, S=O, SO₂, N(R¹), P(R¹) oder P(=O)R¹.

8. Verbindungen nach einem oder mehreren der Ansprüche 1 bis 7, **dadurch gekennzeichnet, dass** Reste R¹, die an die Brücken X gebunden sind, gleich oder verschieden sind und ausgewählt sind aus H, geradkettigen Alkylgruppen mit 1 bis 5 C-Atomen oder verzweigten Alkylgruppen mit 3 bis 5 C-Atomen, wobei eine oder mehrere nicht benachbarte CH₂-Gruppen durch -R²C=CR²-, -C≡C- oder -O- ersetzt sein können und wobei ein oder mehrere H-Atome durch F ersetzt sein können, oder Arylgruppen mit 6 bis 16 C-Atomen oder Heteroarylgruppen mit 2 bis 16 C-Atomen, die jeweils mit einem oder mehreren Resten R² substituiert sein können, oder eine Kombination aus zwei oder drei dieser Systeme; dabei können zwei der Reste R¹, die an dasselbe Brückenatom gebunden sind, auch miteinander ein Ringsystem bilden.

9. Polymere, Oligomere oder Dendrimere enthaltend eine oder mehrere Verbindungen nach einem oder mehreren der Ansprüche 1 bis 8, **dadurch gekennzeichnet, dass** ein oder mehrere Reste R¹ Bindungen zum Polymer, Oligomer oder Dendrimer darstellen.

10. Verfahren zur Herstellung von Verbindungen nach einem oder mehreren der Ansprüche 1 bis 8, umfassend die Schritte:
a) Kupplung eines funktionalisierten Naphthalins mit einem tetrafunktionalisierten Benzolderivat;
b) Ringschluss unter Säurekatalyse; und
c) Funktionalisierung des durch die Schritte a) und b) erhaltenen Grundkörpers durch Bromierung, gefolgt von Hartwig-Buchwald-Kupplung mit einem aromatischen Amin oder Suzuki-Kupplung mit einer Arylboronsäure bzw. einem Arylboronsäurederivat.

11. Verwendung von Verbindungen nach einem oder mehreren der Ansprüche 1 bis 9 in organischen elektronischen Vorrichtungen.

12. Organische elektronische Vorrichtung, enthaltend Anode, Kathode und mindestens eine organische Schicht, enthaltend mindestens eine Verbindung nach einem oder mehreren der Ansprüche 1 bis 9.

13. Organische elektronische Vorrichtung nach Anspruch 12, ausgewählt aus organischen Elektrolumineszenzvorrichtungen (OLED, PLED), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Laserdioden (O-Laser) und organischen Photorezeptoren.

14. Organische Elektrolumineszenzvorrichtung nach Anspruch 13, enthaltend Kathode, Anode, eine oder mehrere emittierende Schichten und gegebenenfalls weitere Schichten, gewählt aus Lochinjektionsschicht, Lochtransportschicht, Elektronentransportschicht, Elektroneninjektionsschicht und/oder Charge-Generation Layer.

15. Organische Elektrolumineszenzvorrichtung nach Anspruch 14, **dadurch gekennzeichnet, dass** ein oder mehrere Substituenten R¹ aus einfachen oder kondensierten Aryl- oder Heteroarylgruppen gewählt sind und die Verbindung nach einem oder mehreren der Ansprüche 1 bis 8 als Host für einen fluoreszierenden Dotanden eingesetzt wird und/oder dass ein oder mehrere Substituenten R¹ und/oder Brücken X mindestens eine Gruppe C=O, P(=O) und/oder SO₂ enthalten und die Verbindung nach einem oder mehreren der Ansprüche 1 bis 8 als Matrix für phosphoreszierende Dotanden eingesetzt wird und/oder dass ein oder mehrere Substituenten R¹ mindestens eine Vinylaryl-Einheit, mindestens eine Vinylarylamin-Einheit und/oder mindestens eine Arylamino-Einheit enthalten und die Verbindungen nach einem oder mehreren der Ansprüche 1 bis 8 als emittierende Materialien eingesetzt werden und/oder dass ein oder mehrere Substituenten R¹ für eine Gruppe N(Ar)₂ stehen und die Verbindung nach einem oder mehreren der Ansprüche 1 bis 8 gegebenenfalls mit Elektronenakzeptor-Verbindungen dotiert sein kann und dass sie als Lochtransportmaterial oder als Lochinjektionsmaterial eingesetzt wird, bevorzugt in einer Lochtransport- oder in einer Lochinjektionsschicht, und/oder dass ein oder mehrere Substituenten R¹ mindestens eine Einheit C=O, P(=O) und/oder SO₂ enthalten und die Verbindungen nach einem oder mehreren der Ansprüche 1 bis 8 gegebenenfalls mit einer Elektronendonator-Verbindung dotiert sein kann und sie als Elektronentransportmaterial eingesetzt wird.

## Claims

1. Compounds of the formula (1) to formula (4), 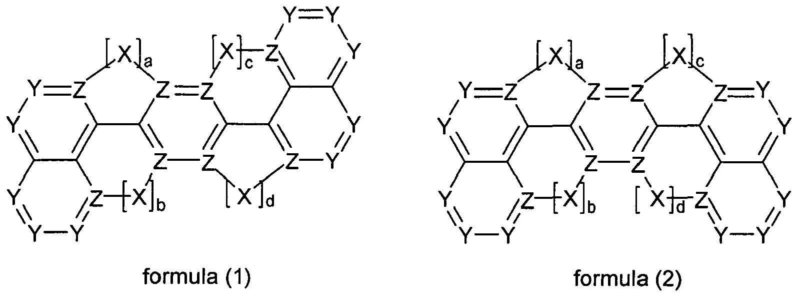 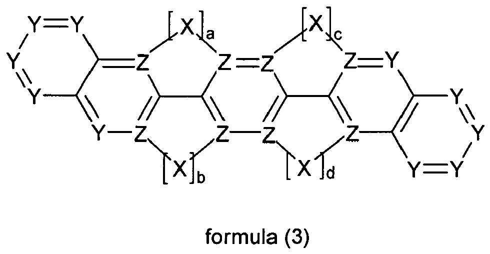 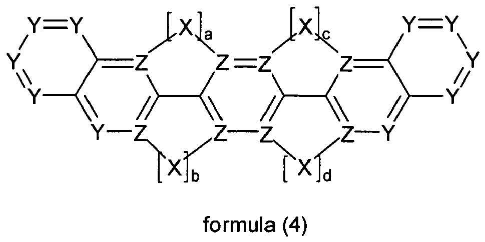 where the following applies to the symbols and indices:
Y is on each occurrence, identically or differently, CR¹ or N;
Z is equal to C if a bridge X is bonded to the group Z and is equal to Y if no bridge X is bonded to the group Z;
X is on each occurrence, identically or differently, a divalent bridge selected from B(R¹), C(R¹)₂, Si(R¹)₂, C=O, C=NR¹, C=C(R¹)₂, O, S, S=O, SO₂, N(R¹), P(R¹) and P(=O)R¹;
R¹ is on each occurrence, identically or differently, H, F, Cl, Br, I, N(Ar)₂, C(=O)Ar, P(=O)Ar₂, S(=O)Ar, S(=O)₂Ar, CR²=CR²Ar, CN, NO₂, Si(R²)₃, B(OR²)₂, OSO₂R², a straight-chain alkyl, alkoxy or thioalkoxy group having 1 to 40 C atoms or a branched or cyclic alkyl, alkoxy or thioalkoxy group having 3 to 40 C atoms, each of which may be substituted by one or more radicals R² and in which one or more non-adjacent CH₂ groups may be replaced by R²C=CR², C=C, Si(R²)₂, Ge(R²)₂, Sn(R²)_{2,} C=O, C=S, C=Se, C=NR², P(=O)(R²), SO, SO₂, NR², O, S or CONR² and in which one or more H atoms may be replaced by F, Cl, Br, I, CN or NO₂, or an aromatic or heteroaromatic ring system having 5 to 40 aromatic ring atoms, which may in each case be substituted by one or more radicals R², or an aryloxy or heteroaryloxy group having 5 to 40 aromatic ring atoms, which may be substituted by one or more radicals R², or a combination of these systems; two or more adjacent substituents R¹ here may also form a mono- or polycyclic aliphatic or aromatic ring system with one another;
Ar is on each occurrence, identically or differently, an aromatic or heteroaromatic ring system having 5-30 aromatic ring atoms, which may be substituted by one or more non-aromatic radicals R¹; two radicals Ar here on the same nitrogen or phosphorus atom may also be linked to one another by a single bond or a bridge X;
R² is on each occurrence, identically or differently, H or an aliphatic, aromatic and/or heteroaromatic hydrocarbon radical having 1 to 20 C atoms, in which, in addition, H atoms may be replaced by F; two or more adjacent substituents R² here may also form a mono- or polycyclic aliphatic or aromatic ring system with one another;
a, b, c, d are on each occurrence, identically or differently, 0 or 1, with the proviso that a + b = 1 or 2 and c + d = 1 or 2, where a = 0 and b = 0 and c = 0 and d = 0 in each case means that the corresponding bridge X is not present;
with the exception of the following compounds:

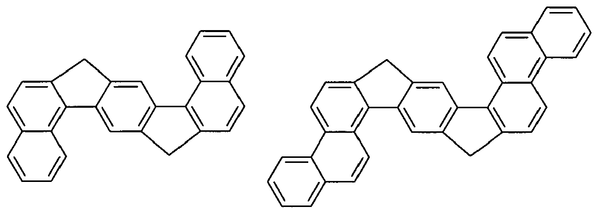

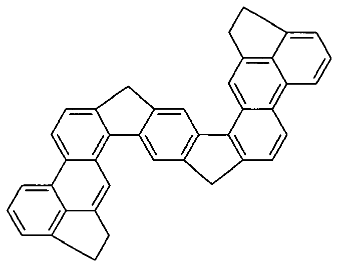

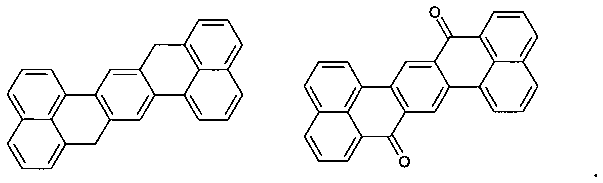

2. Compounds according to Claim 1, **characterised in that**, for the indices a, b, c and d, a + b = 1 and c + d = 1.

3. Compounds according to Claim 1 or 2 selected from the compounds of the formulae (5), (6), (7), (8), (9), (10), (11), (12), (13), (14), (15) and (16), 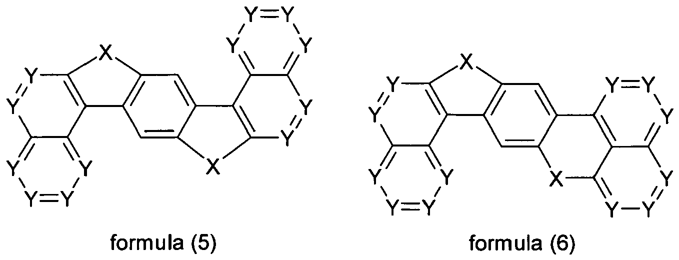 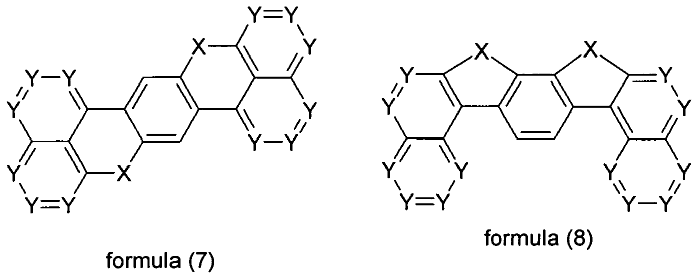 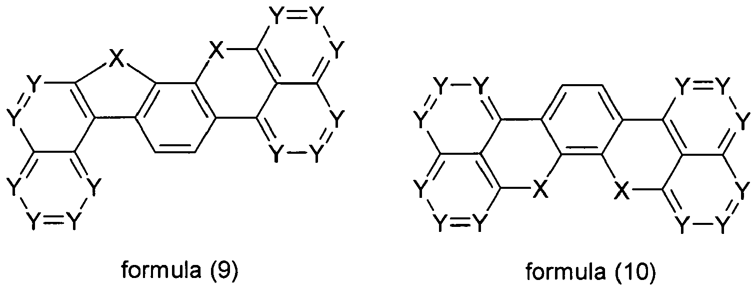 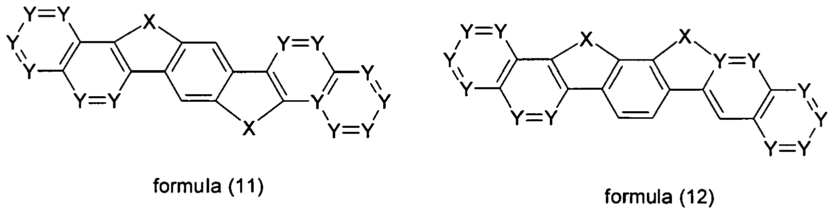 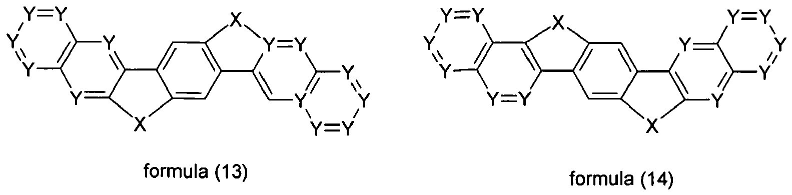 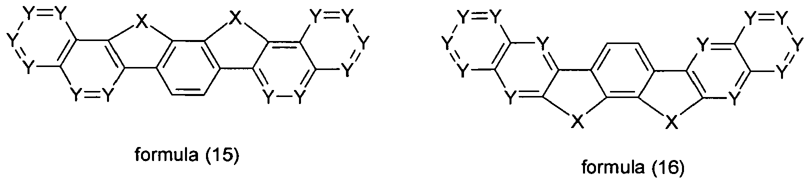 where the symbols X and Y have the same meaning as described in Claim 1.

4. Compounds according to one or more of Claims 1 to 3, **characterised in that** the symbol Y stands for nitrogen a total of 0, 1, 2, 3 or 4 times, where the other symbols Y stand for CR¹.

5. Compounds according to one or more of Claims 1 to 4, selected from the formulae (5a), (6a), (7a), (8a), (9a), (10a) and (11a), 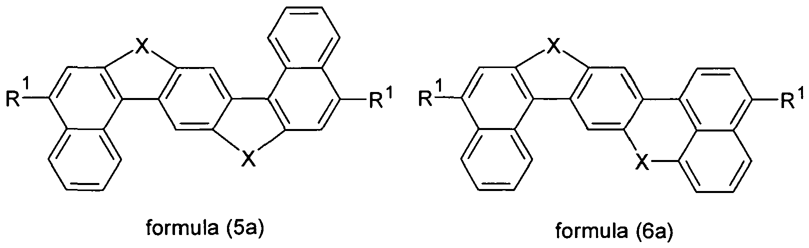 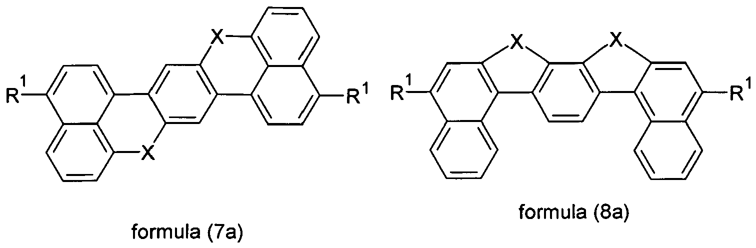 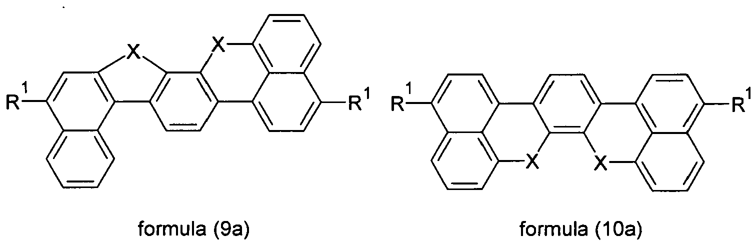 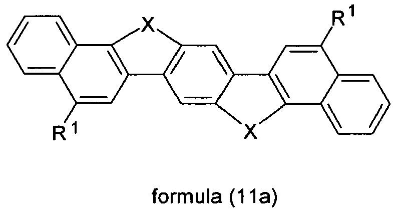 where the symbols X and R¹ have the same meaning as described in Claim 1.

6. Compounds according to one or more of Claims 1 to 5, **characterised in that** the symbol R¹, identically or differently on each occurrence, stands for H, F, Br, C(=O)Ar, P(=O)Ar₂, CR²=CR²Ar, a straight-chain alkyl group having 1 to 5 C atoms or a branched alkyl group having 3 to 5 C atoms, in which one or more non-adjacent CH₂ groups may be replaced by -R²C=CR²-, -C≡C- or -O- and in which one or more H atoms may be replaced by F, or an aryl group having 6 to 16 C atoms or a heteroaryl group having 2 to 16 C atoms or a spirobifluorene group, each of which may be substituted by one or more radicals R², or a combination of two or three of these systems, and/or **in that** at least one symbol R¹ stands for a group N(Ar)₂ of the formula (17) or (18), 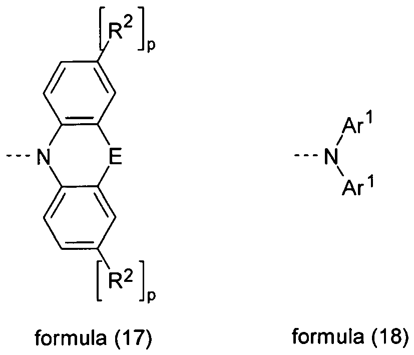 where R² has the meaning mentioned in Claim 1, and furthermore:
E stands for a single bond, O, S, N(R²) or C(R²)₂;
Ar¹ is, identically or differently on each occurrence, an aryl or heteroaryl group having 5 to 20 aromatic ring atoms or a triarylamine group having 15 to 30 aromatic ring atoms, each of which may be substituted by one or more radicals R², preferably an aryl or heteroaryl group having 6 to 14 aromatic ring atoms or a triarylamine group having 18 to 22 aromatic ring atoms, each of which may be substituted by one or more radicals R²;
p is on each occurrence, identically or differently, 0 or 1.

7. Compounds according to one or more of Claims 1 to 6, **characterised in that** the symbol X is on each occurrence, identically or differently, a divalent bridge selected from C(R¹)₂, C=O, C=NR¹, O, S, S=O, SO₂, N(R¹), P(R¹) and P(=O)R¹.

8. Compounds according to one or more of Claims 1 to 7, **characterised in that** radicals R¹ which are bonded to the bridges X are identical or different and are selected from H, straight-chain alkyl groups having 1 to 5 C atoms or branched alkyl groups having 3 to 5 C atoms, in which one or more non-adjacent CH₂ groups may be replaced by -R²C=CR²-, -C≡C- or -O- and in which one or more H atoms may be replaced by F, or aryl groups having 6 to 16 C atoms or heteroaryl groups having 2 to 16 C atoms, each of which may be substituted by one or more radicals R², or a combination of two or three of these systems; two of the radicals R¹ which are bonded to the same bridge atom here may also form a ring system with one another.

9. Polymers, oligomers or dendrimers comprising one or more compounds according to one or more of Claims 1 to 8, **characterised in that** one or more radicals R¹ represent bonds to the polymer, oligomer or dendrimer.

10. Process for the preparation of compounds according to one or more of Claims 1 to 8, comprising the steps:
a) coupling of a functionalised naphthalene to a tetrafunctionalised benzene derivative;
b) ring closure with acid catalysis; and
c) functionalisation of the parent structure obtained by steps a) and b) by bromination, followed by Hartwig-Buchwald coupling to an aromatic amine or Suzuki coupling to an arylboronic acid or an arylboronic acid derivative.

11. Use of compounds according to one or more of Claims 1 to 9 in organic electronic devices.

12. Organic electronic device comprising anode, cathode and at least one organic layer comprising at least one compound according to one or more of Claims 1 to 9.

13. Organic electronic device according to Claim 12 selected from organic electroluminescent devices (OLEDs, PLEDs), organic field-effect transistors (O-FETs), organic thin-film transistors (O-TFTs), organic light-emitting transistors (O-LETs), organic integrated circuits (O-ICs), organic solar cells (O-SCs), organic field-quench devices (O-FQDs), light-emitting electrochemical cells (LECs), organic laser diodes (O-lasers) and organic photoreceptors.

14. Organic electroluminescent device according to Claim 13 comprising cathode, anode, one or more emitting layers and optionally further layers selected from hole-injection layer, hole-transport layer, electron-transport layer, electron-injection layer and/or charge-generation layer.

15. Organic electroluminescent device according to Claim 14, **characterised in that** one or more substituents R¹ are selected from simple or condensed aryl or heteroaryl groups and the compound according to one or more of Claims 1 to 8 is employed as host for a fluorescent dopant and/or **in that** one or more substituents R¹ and/or bridges X contain at least one group C=O, P(=O) and/or SO₂ and the compound according to one or more of Claims 1 to 8 is employed as matrix for phosphorescent dopants and/or **in that** one or more substituents R¹ contain at least one vinylaryl unit, at least one vinylarylamine unit and/or at least one arylamino unit and the compounds according to one or more of Claims 1 to 8 are employed as emitting materials and/or **in that** one or more substituents R¹ stand for a group N(Ar)₂ and the compound according to one or more of Claims 1 to 8 may optionally be doped with electron-acceptor compounds and **in that** it is employed as hole-transport material or as hole-injection material, preferably in a hole-transport or hole-injection layer, and/or **in that** one or more substituents R¹ contain at least one unit C=O, P(=O) and/or SO₂ and the compounds according to one or more of Claims 1 to 8 may optionally be doped with an electron-donor compound and are employed as electron-transport material.

## Revendications

1. Composés de la formule (1) à la formule (4), 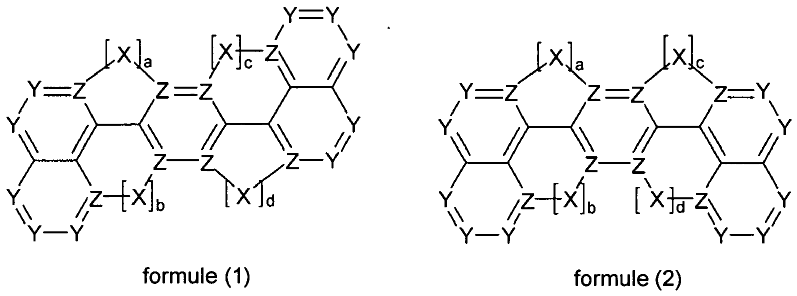 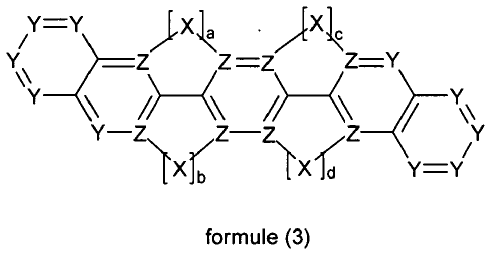 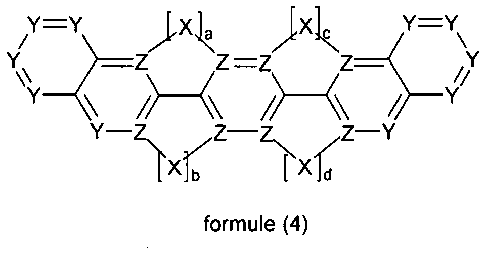 où ce qui suit s'applique aux symboles et indices :
Y est, pour chaque occurrence, de manière identique ou différente, CR¹ ou N ;
Z est égal à C si un pont X est lié au groupe Z et est égal à Y si aucun pont X n'est lié au groupe Z ;
X est, pour chaque occurrence, de manière identique ou différente, un pont divalent choisi parmi B(R¹), C(R¹)₂, Si(R¹)₂, C=O, C=NR¹, C=C(R¹)₂, O, S, S=O, SO₂, N(R¹), P(R¹) et P(=O)R^{1 ;}
R¹ est, pour chaque occurrence, de manière identique ou différente, H, F, Cl, Br, I, N(Ar)₂, C(=O)Ar, P(=O)Ar₂, S(=O)Ar, S(=O)₂Ar, CR²=CR²Ar, CN, NO₂, Si(R²)₃, B(OR²)₂, OSO₂R², un groupe alkyle, alcoxy ou thioalcoxy en chaîne droite comportant de 1 à 40 atomes de C ou un groupe alkyle, alcoxy ou thioalcoxy ramifié ou cyclique comportant de 3 à 40 atomes de C, dont chacun peut être substitué par un ou plusieurs radicaux R² et où un ou plusieurs groupes CH₂ non adjacents peuvent être remplacés par R²C=CR², C≡C, Si(R²)₂, Ge(R²)_{2,} Sn(R²)₂, C=O, C=S, C=Se, C=NR², P(=O)(R²), SO, SO₂, NR², O, S ou CONR² et où un ou plusieurs atomes de H peuvent être remplacés par F, Cl, Br, I, CN ou NO₂, ou un système de cycle aromatique ou hétéroaromatique comportant de 5 à 40 atomes de cycle aromatique, lesquels peuvent dans chaque cas être substitués par un ou plusieurs radicaux R², ou un groupe aryloxy ou hétéroaryloxy comportant de 5 à 40 atomes de cycle aromatique, lesquels peuvent être substitués par un ou plusieurs radicaux R², ou une combinaison de ces systèmes ; deux substituants adjacents ou plus R¹ peuvent également ici former un système de cycle aliphatique ou aromatique monocyclique ou polycyclique les uns avec les autres ;
Ar est, pour chaque occurrence, de manière identique ou différente, un système de cycle aromatique ou hétéroaromatique comportant de 5 à 30 atomes de cycle aromatique, lesquels peuvent être substitués par un ou plusieurs radicaux non aromatiques R¹; deux radicaux Ar ici sur le même atome d'azote ou de phosphore peuvent être également liés l'un à l'autre par une liaison simple ou un pont X ;
R² est, pour chaque occurrence, de manière identique ou différente, H ou un radical hydrocarbone aliphatique, aromatique et/ou hétéroaromatique comportant de 1 à 20 atomes de C, où, en plus, des atomes de H peuvent être remplacés par F ; deux substituants R² adjacents ou plus peuvent ici également former un système de cycle aliphatique ou aromatique monocyclique ou polycyclique les uns avec les autres ;
a, b, c, d sont, pour chaque occurrence, de manière identique ou différente, 0 ou 1, étant entendu que a + b = 1 ou 2 et c + d=1 ou 2, où a = 0 et b = 0 et c = 0 et d = 0 dans chaque cas signifie que le pont correspondant X n'est pas présent ;
moyennant l'exception des composés qui suivent :

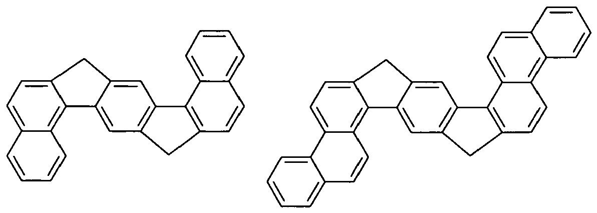

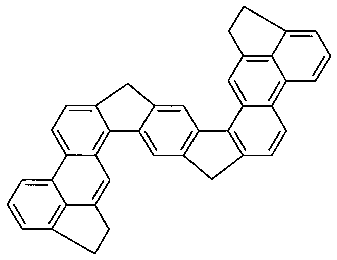

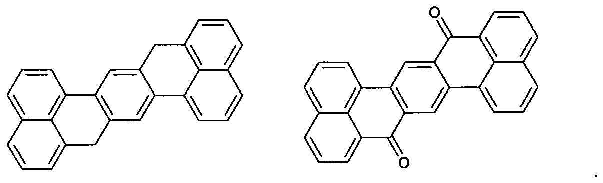

2. Composés selon la revendication 1, **caractérisés en ce que**, pour les indices a, b, c et d, a + b = 1 et c + d = 1.

3. Composés selon la revendication 1 ou 2 choisis parmi les composés des formules (5), (6), (7), (8), (9), (10), (11), (12), (13), (14), (15) et (16), 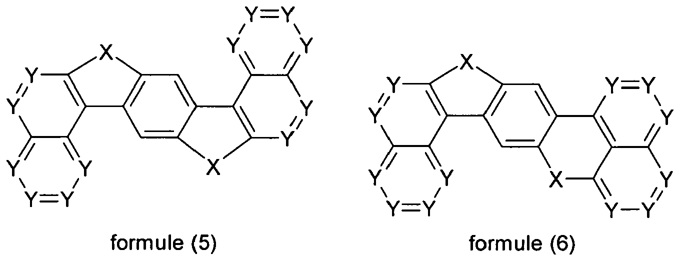 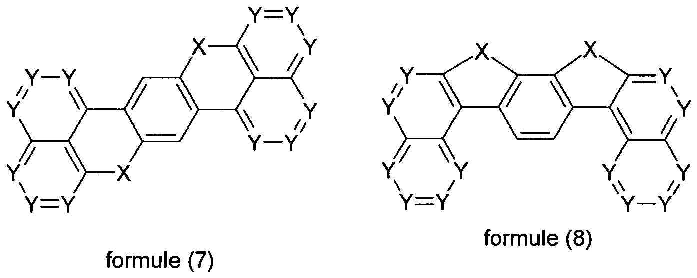 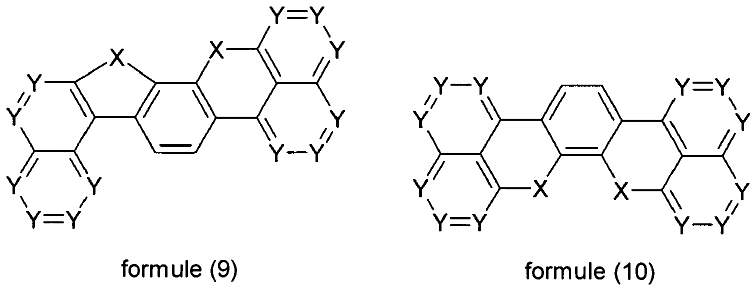 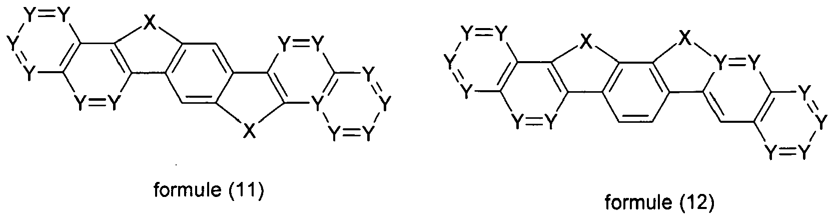 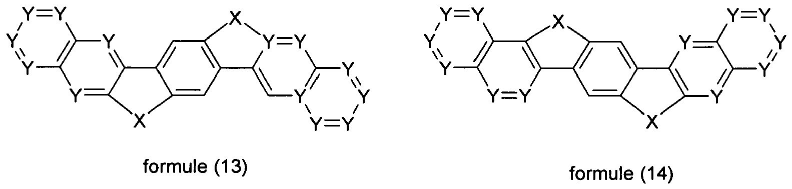 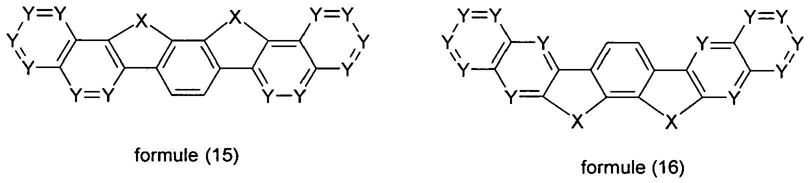 où les symboles X et Y présentent la même signification que décrit selon la revendication 1.

4. Composés selon une ou plusieurs des revendications 1 à 3, **caractérisés en ce que** le symbole Y représente azote un total de 0, 1, 2, 3 ou 4 fois, où les autres symboles Y représentent CR¹.

5. Composés selon une ou plusieurs des revendications 1 à 4, choisis parmi les formules (5a), (6a), (7a), (8a), (9a), (10a) et (11 a), 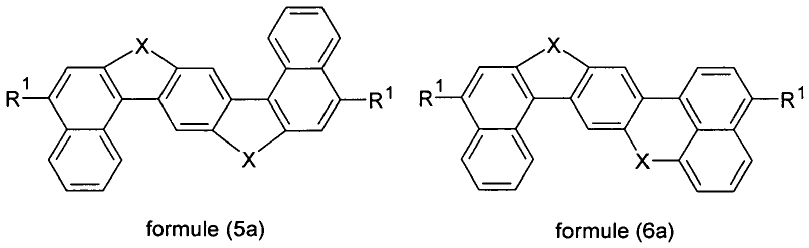 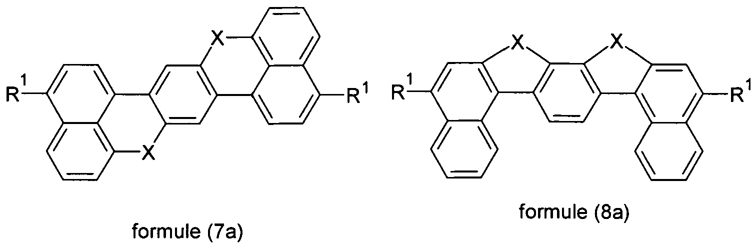 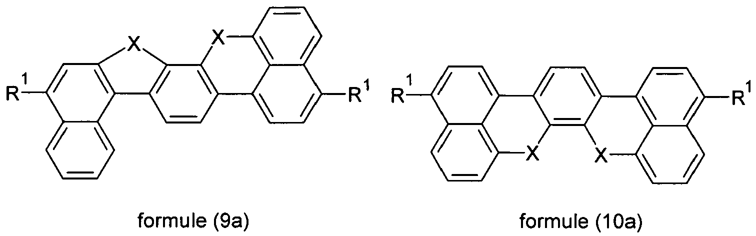 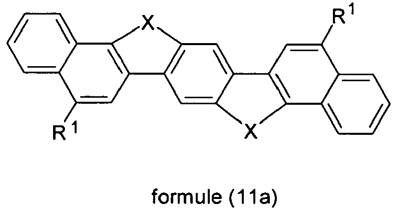 où les symboles X et R¹ présentent la même signification que décrit selon la revendication 1.

6. Composés selon une ou plusieurs des revendications 1 à 5, **caractérisés en ce que** le symbole R¹, de manière identique ou différente pour chaque occurrence, représente H, F, Br, C(=O)Ar, P(=O)Ar₂, CR²=CR²Ar, un groupe alkyle en chaîne droite comportant de 1 à 5 atomes de C ou un groupe alkyle ramifié comportant de 3 à 5 atomes de C, où un ou plusieurs groupes CH₂ non adjacents peuvent être remplacés par -R²C=CR²-, -C=C- ou -O- et où un ou plusieurs atomes de H peuvent être remplacés par F, ou un groupe aryle comportant de 6 à 16 atomes de C ou un groupe hétéroaryle comportant de 2 à 16 atomes de C ou un groupe spirobifluorène, dont chacun peut être substitué par un ou plusieurs radicaux R², ou une combinaison de deux ou trois de ces systèmes, et/ou **en ce qu'**au moins un symbole R¹ représente un groupe N(Ar)₂ de la formule (17) ou (18), 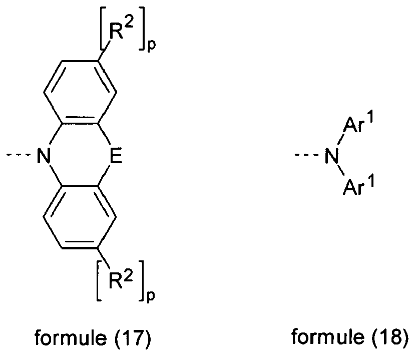 où R² présente la signification mentionnée dans la revendication 1, et en outre :
E représente une liaison simple, O, S, N(R²) ou C(R²)_{2 ;}
Ar¹ est, de manière identique ou différente pour chaque occurrence, un groupe aryle ou hétéroaryle comportant de 5 à 20 atomes de cycle aromatique ou un groupe triarylamine comportant de 15 à 30 atomes de cycle aromatique, dont chacun peut être substitué par un ou plusieurs radicaux R², de préférence un groupe aryle ou hétéroaryle comportant de 6 à 14 atomes de cycle aromatique ou un groupe triarylamine comportant de 18 à 22 atomes de cycle aromatique, dont chacun peut être substitué par un ou plusieurs radicaux R² ;
p est, pour chaque occurrence, de manière identique ou différente, 0 ou 1.

7. Composés selon une ou plusieurs des revendications 1 à 6, **caractérisés en ce que** le symbole X est, pour chaque occurrence, de manière identique ou différente, un pont divalent choisi parmi C(R¹)₂, C=O, C=NR¹, O, S, S=O, SO₂, N(R¹), P(R¹) et P(=O)R¹.

8. Composés selon une ou plusieurs des revendications 1 à 7, **caractérisés en ce que** des radicaux R¹ qui sont liés aux ponts X sont identiques ou différents et sont choisis parmi H, des groupes alkyle en chaîne droite comportant de 1 à 5 atomes de C ou des groupes alkyle ramifiés comportant de 3 à 5 atomes de C, où un ou plusieurs groupes CH₂ non adjacents peuvent être remplacés par -R²C=CR²-, -C≡C- ou -O- et où un ou plusieurs atomes de H peuvent être remplacés par F, ou des groupes aryle comportant de 6 à 16 atomes de C ou des groupes hétéroaryle comportant de 2 à 16 atomes de C, dont chacun peut être substitué par un ou plusieurs radicaux R², ou une combinaison de deux ou trois de ces systèmes ; deux des radicaux R¹ qui sont liés au même atome de pont peuvent également ici former un système de cycle l'un avec l'autre.

9. Polymères, oligomères ou dendrimères comprenant un ou plusieurs composés selon une ou plusieurs des revendications 1 à 8, **caractérisés en ce qu'**un ou plusieurs radicaux R¹ représentent des liaisons sur le polymère, l'oligomère ou le dendrimère.

10. Procédé pour la préparation de composés selon une ou plusieurs des revendications 1 à 8, comprenant les étapes de :
a) couplage d'un naphtalène fonctionnalisé à un dérivé de benzène tétrafonctionnalisé ;
b) fermeture de cycle avec une catalyse par acide ; et
c) fonctionnalisation de la structure parente obtenue au moyen des étapes a) et b) par bromination, suivie par un couplage de Hartwig-Buchwald sur un amine aromatique ou par un couplage de Suzuki sur un acide arylboronique ou un dérivé d'acide arylboronique.

11. Utilisation de composés selon une ou plusieurs des revendications 1 à 9 dans des dispositifs électroniques organiques.

12. Dispositif électronique organique comprenant une anode, une cathode et au moins une couche organique comprenant au moins un composé selon une ou plusieurs des revendications 1 à 9.

13. Dispositif électronique organique selon la revendication 12 choisi parmi des dispositifs électroluminescents organiques (OLED, PLED), des transistors à effet de champ organiques (O-FET), des transistors à film mince organiques (O-TFT), des transistors émetteurs de lumière organiques (O-LET), des circuits intégrés organiques (O-IC), des cellules solaires organiques (O-SC), des dispositifs à extinction de champ organiques (O-FQD), des cellules électrochimiques émettrices de lumière (LEC), des diodes laser organiques (O-laser) et des photorécepteurs organiques.

14. Dispositif électroluminescent organique selon la revendication 13 comprenant une cathode, une anode, une ou plusieurs couches émettrices et en option, d'autres couches choisies parmi une couche d'injection de trous, une couche de transport de trous, une couche de transport d'électrons, une couche d'injection d'électrons et/ou une couche de génération de charges.

15. Dispositif électroluminescent organique selon la revendication 14, **caractérisé en ce qu'**un ou plusieurs substituants R¹ sont choisis parmi des groupes aryle ou hétéroaryle simples ou condensés et le composé selon une ou plusieurs des revendications 1 à 8 est utilisé en tant qu'hôte pour un dopant fluorescent et/ou **en ce qu'**un ou plusieurs substituants R¹ et/ou ponts X contiennent au moins un groupe C=O, P(=O) et/ou SO₂ et le composé selon une ou plusieurs des revendications 1 à 8 est utilisé en tant que matrice pour des dopants phosphorescents et/ou **en ce qu'**un ou plusieurs substituants R¹ contiennent au moins une unité vinylaryle, au moins une unité vinylarylamine et/ou au moins une unité arylamino et les composés selon une ou plusieurs des revendications 1 à 8 sont utilisés en tant que matériaux émetteurs et/ou **en ce qu'**un ou plusieurs substituants R¹ représentent un groupe N(Ar)₂ et le composé selon une ou plusieurs des revendications 1 à 8 peut en option être dopé avec des composés accepteurs d'électrons et **en ce qu'**il est utilisé en tant que matériau de transport de trous ou en tant que matériau d'injection de trous, de préférence dans une couche de transport de trous ou une couche d'injection de trous, et/ou **en ce qu'**un ou plusieurs substituants R¹ contiennent au moins une unité C=O, P(=O) et/ou SO₂ et les composés selon une ou plusieurs des revendications 1 à 8 peuvent en option être dopés avec un composé donneur d'électrons et sont utilisés en tant que matériau de transport d'électrons.
